(19) Europäisches Patentamt
European Patent Office
Office européen des brevets

(11) Veröffentlichungsnummer: **0 533 056 A2**

(12) # EUROPÄISCHE PATENTANMELDUNG

(21) Anmeldenummer: **92115497.7**

(22) Anmeldetag: **10.09.92**

(51) Int. Cl.5: **C07C 217/34**, C07C 217/32, A61K 31/13, C07D 211/58, A61K 31/44

(30) Priorität: **14.09.91 DE 4130708**

(43) Veröffentlichungstag der Anmeldung: **24.03.93 Patentblatt 93/12**

(84) Benannte Vertragsstaaten: **AT BE CH DE DK ES FR GB GR IE IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Kirsch, Reinhard, Dr.**
**Johannesallee 18**
**W-6230 Frankfurt am Main 80(DE)**
Erfinder: **Hänel, Heinz, Dr.**
**Taunusstrasse 148**
**W-6370 Oberursel(DE)**
Erfinder: **Kottmann, Hariolf, Dr.**
**Am Alten Birnbaum 6**
**W-6238 Hofheim (Ts)(DE)**
Erfinder: **Linkies, Adolf Heinz, Dr.**
**Loreleistrasse 12**
**W-6230 Frankfurt am Main 80(DE)**
Erfinder: **Reuschling, Dieter Bernd, Dr.**
**Beethovenstrasse 27**
**W-6308 Butzbach(DE)**
Erfinder: **Wehner, Volkmar, Dr.**
**Lindenstrasse 1**
**W-8741 Sandberg 2(DE)**

(54) **Substituierte Aminopropane, Verfahren zu ihrer Herstellung und ihre Verwendung als Antimykutiku.**

(57) Verbindungen der Formel I

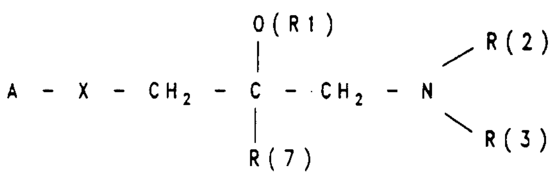

dienen zur Behandlung und zur Prophylaxe von Pilzenkrankungen. Sie werden durch Umsetzung von II

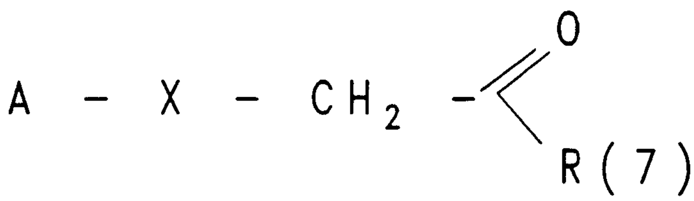

II

mit III

EP 0 533 056 A2

$$\begin{array}{l} (H_3C)_2S\ =\ CH_2, \\ \qquad \| \\ \quad\ (O)_p \end{array} \qquad (III)$$

zu IV

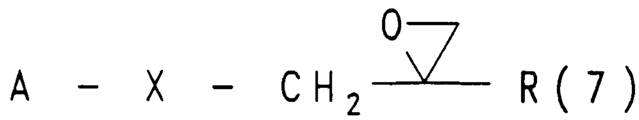

(IV)

und Umsetzung mit einem Nucleophil M-NR(2)R(3) erhalten.

Die Erfindung betrifft substituierte Aminopropane der Formel I, Verfahren zu ihrer Herstellung sowie solche Verbindungen enthaltende pharmazeutische Zusammensetzungen und ihre Verwendung als Pharmazeutika, insbesondere als Wachstumshemmer der pathogenen Phase dimorpher Hefezellen und als Antimykotika, wobei die Verwendung der erfindungsgemäßen Verbindungen sowohl in der Prophylaxe als auch in der Therapie stattfinden kann.

In der Deutschen Offenlegungsschrift DE 3330005 A1 werden Verbindungen der Formel A

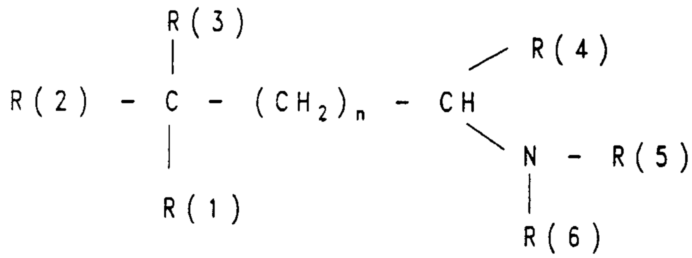

A

genannt. In dieser Offenlegungsschrift ist R(3) allgemein als Aryl, Alkyl oder Cycloalkyl, wobei die Reste jeweils substituiert sein können, sowie Wasserstoff definiert. Es findet sich jedoch in der Beschreibung und in den Beispielen keinerlei Hinweis auf Verbindungen mit R(3) gleich Aryl, Alkyl oder Cycloalkyl, sondern es sind nur Weinsäurederivate von solchen Verbindungen mit R(3) gleich Wasserstoff beschrieben.

Nicht bekannt sind hingegen Verbindungen der Formel I, in denen die Aminkomponente aliphatische, cycloaliphatische oder aromatische Reste trägt und gleichzeitig der Substituent R(7) in Formel I eine über ein Kohlenstoffatom gebundene Gruppe ist, ebenso wie deren Verwendung als Hemmstoffe der Exoenzyme (Pathogenitätsfaktoren) von Pilzen und als Antimykotika.

Die Erfindung betrifft daher substituierte Aminopropane der Formel I

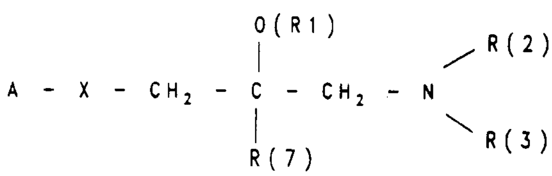

I

deren Herstellung und deren Verwendung als Antimykotika sowie als Wachstumshemmer der pathogenen Phase dimorpher Hefezellen. Die Verbindungen dienen daher zur Prophylaxe und Behandlung von Pilzerkrankungen. Die Erfindung betrifft ferner diese Verbindungen enthaltende pharmazeutische Zusammensetzungen sowie ihre Salze mit pharmazeutisch akzeptablen Säuren und ihre physiologisch hydrolysierbaren Derivate.

Alkyl-, Alkoxy-, Alkylthio-, Alkylen- und Alkenylgruppen in jeder Form sind, soweit nicht ausdrücklich anders erwähnt, geradkettig oder verzweigt; Alkenylketten sind ein- oder mehrfach ungesättigt. Aryl bedeutet Phenyl oder Naphthyl. Als Arylrest können aber auch Phenanthryl-, Fluorenyl und Anthracenyl fungieren.

In Formel I bedeuten:

R(1): H, ($C_1$- $C_{10}$)-Alkyl, ($C_2$-$C_{10}$)-Alkenyl, Benzyl, [unsubstituiert oder ein-oder mehrfach substituiert durch F, Cl, Br, $CF_3$, ($C_1$-$C_4$)-Alkyl, $OCH_3$, O-Phenyl oder Phenyl], C(O)-($C_1$-$C_8$)-Alkyl, C(O)-phenyl

R(2): H, ($C_1$-$C_{18}$)-Alkyl, ($C_1$-$C_6$)-Alkyl-oxy-($C_1$-$C_6$)-Alkyl, ($C_1$-$C_6$)-Alkyl-thio-phenyl, ($C_1$-$C_6$)-Alkyl-oxy-phenyl, ($C_2$-$C_{18}$)-Alkenyl, ($C_3$-$C_{12}$)-Cycloalkyl (mono-, bi- oder multicyclisch, wie Norbornyl, Adamantyl, Dekahydronaphthyl), Phenyl-($C_1$-$C_6$)-Alkyl, wobei die Alkylkette unsubstituiert oder mit OH ein- oder zweifach substituiert ist), Phenyl-($C_2$-$C_6$)-Alkenyl, Diphenyl-($C_1$-$C_6$-)-Alkyl, Phenyl, wobei die Phenylsysteme unsubstituiert oder ein- oder mehrfach substituiert sind durch Substituenten aus der Gruppe F, Cl, Br, ($C_1$-$C_{10}$)-Alkyl, ($C_3$-$C_{10}$)-Cycloalkyl, OH, SH, ($C_1$-$C_{10}$)-Alkoxy, ($C_1$-$C_4$)-Alkylendioxy, ($C_1$-$C_{10}$)-Alkylthio, Dimethylaminoethoxy, ($C_1$-$C_4$)-Alkoxycarbonylmethoxy, Phenoxy, Phenyl, Benzyl, Phenethyl, Thiophenyl, $C_aF_{2a+1}$ (mit a gleich 1 - 6).

oder

R(2) Indol-3-yl-($C_1$-$C_4$)-Alkyl-, Thienyl-, Thienylmethylrest, wobei der Thienylring unsubstituiert oder mit F, Cl, ($C_1$-$C_4$)-Alkyl, O($C_1$-$C_4$)-Alkyl substituiert ist,

oder

R(2) Piperidin-4-yl, unsubstituiert oder mit 1 bis 4 Methylgruppen substituiert und am Piperidin N-Atom ein Wasserstoff, eine Acetyl-, Phenyl- oder Benzylgruppe tragend,

R(3) ist wie R(2) definiert, wobei R(2) und R(3) jeweils gleiche oder verschiedene Bedeutung aufweisen, oder

R(2) bildet mit R(3) eine Kette $-(CH_2)_m-$ mit m gleich 4 - 6, in welcher eine $CH_2$-Gruppe durch Sauerstoff, Schwefel ersetzt sein kann,

A ($C_1$-$C_{18}$)-Alkyl, ($C_2$-$C_{18}$)-Alkenyl oder eine Gruppe

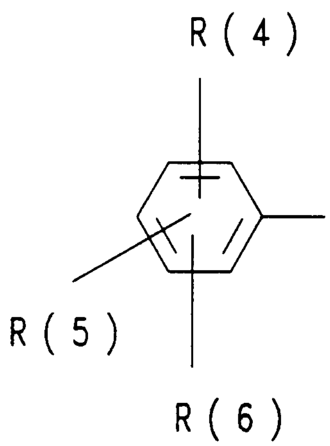

,

worin die Reste R(4), R(5) und R(6) die folgende Bedeutung haben:

R(4) H, ($C_1$-$C_{18}$)-Alkyl, ($C_2$-$C_{18}$)-Alkenyl, ($C_3$-$C_{20}$)-Cycloalkyl (mono-, bi-oder multicyclisch), Y-($C_1$-$C_{20}$)-Alkyl, Y-($C_2$-$C_{18}$)-Alkenyl, Y-($C_3$-$C_{20}$)-Cycloalkyl (mono-, bi- oder multicyclisch), Phenyl, Y-Phenyl, Phenyl-($C_1$-$C_6$)-Alkyl, Phenyl-($C_1$-$C_4$)-Alkoxy, Biphenylyl, F, Cl, Br, $C_aF_{2a+1}$ (mit a gleich 1 bis 8), $CCl_3$, YH, Naphthyl, CN, $NO_2$, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy, CN, $-O-(CH_2)_{1-2}-O-$,

mit Y gleich Sauerstoff oder Schwefel, SO, $SO_2$,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)_r$-Kette mit r gleich 3 oder 4, oder eine $O(CH_2)_{1-2}$-O-Kette,

R(6) H, ($C_1$-$C_{15}$)-Alkyl, ($C_2$-$C_{15}$)-Alkenyl, Y-($C_1$-$C_{15}$)-Alkyl, Y-($C_2$-$C_{15}$)-Alkenyl, Phenyl, Y-Phenyl, Benzyl, Biphenylyl, F, Cl, Br, I, $C_aF_{2a+1}$ (mit a gleich 1 - 8), $CCl_3$, Naphthyl, YH,

X Sauerstoff, Schwefel,

R(7) ($C_1$-$C_{12}$)-Alkyl, ($C_3$-$C_{12}$)-Cycloalkyl (mono-, bi- oder multicyclisch, wie Norbornyl oder Adamantyl), ($C_3$-$C_{12}$)-Alkenyl, Phenyl-thio-phenyl, Phenyl-oxy-phenyl, Biphenylyl, Phenyl-methyl-phenyl, Naphthyl, Phenyl, Thienyl, Pyridyl, wobei die genannten aromatischen und heteroaromatischen Ringsysteme unsubstituiert oder ein- oder mehrfach substituiert sind durch F, Cl, Br, ($C_1$-$C_{18}$)-Alkyl, ($C_1$-$C_{18}$)-Alkoxy, ($C_2$-$C_{18}$)-Alkenyloxy, $CF_3$, $C_2F_5$, CN, ($C_1$-$C_{18}$)-Alkylthio.

Bevorzugt sind Verbindungen der Formel I, worin bedeuten:

R(1): H, ($C_1$- $C_6$)-Alkyl, Benzyl, [unsubstituiert oder ein- oder zweifach substituiert durch F, Cl, Br, $CF_3$, ($C_1$-$C_4$)-Alkyl, $OCH_3$, O-Phenyl oder Phenyl], C(O)-($C_1$-$C_8$)-Alkyl, C(O)-phenyl

R(2): H, ($C_1$-$C_{18}$)-Alkyl, ($C_1$-$C_6$)-Alkyl-oxy-($C_1$-$C_6$)-Alkyl, ($C_1$-$C_6$)-Alkyl-thio-phenyl, ($C_1$-$C_6$)-Alkyl-oxy-phenyl, ($C_2$-$C_{18}$)-Alkenyl, ($C_3$-$C_{12}$)-Cycloalkyl (mono-, bi- oder multicyclisch, wie Norbornyl, Adamantyl, Dekahydronaphthyl), Phenyl-($C_1$-$C_6$)-Alkyl, wobei die Alkylkette unsubstituiert oder mit OH ein- oder zweifach substituiert ist), Phenyl-($C_2$-$C_6$)-Alkenyl, Diphenyl-($C_1$-$C_6$-)-Alkyl, Phenyl, wobei die Phenylsysteme unsubstituiert oder ein- oder mehrfach substituiert sind durch Substituenten aus der Gruppe F, Cl, Br, ($C_1$-$C_{10}$)-Alkyl, ($C_3$-$C_{10}$)-Cycloalkyl, ($C_1$-$C_{10}$)-Alkoxy, ($C_1$-$C_4$)-Alkylendioxy, ($C_1$-$C_4$)-Alkoxycarbonylmethoxy, Phenoxy, Phenyl, Benzyl, Phenethyl, Thiophenyl, $CF_3$.

oder

R(2) Indol-3-yl-($C_1$-$C_4$)-Alkyl-, Thienyl- oder Thienylmethylrest, wobei der Thienylring unsubstituiert oder mit Cl, ($C_1$-$C_4$)-Alkyl, O($C_1$-$C_4$)-Alkyl substituiert ist,

oder

R(2) Piperidin-4-yl, unsubstituiert oder mit 1 bis 4 Methylgruppen substituiert und am Piperidin N-Atom ein Wasserstoff, eine Acetyl-, Phenyl- oder Benzylgruppe tragend,

R(3) ist wie R(2) definiert, wobei R(2) und R(3) jeweils gleiche oder verschiedene Bedeutung aufweisen, oder

A ($C_1$-$C_{18}$)-Alkyl, ($C_2$-$C_{18}$)-Alkenyl oder eine Gruppe

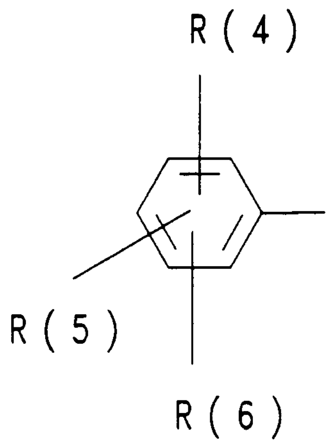

,

worin die Reste R(4), R(5) und R(6) die folgende Bedeutung haben:

R(4) H, ($C_1$-$C_{16}$)-Alkyl, ($C_3$-$C_{16}$)-Alkenyl, ($C_3$-$C_{12}$)-Cycloalkyl (mono-, bi-oder multicyclisch), Y-($C_1$-$C_{16}$)-Alkyl, Y-($C_3$-$C_{16}$)-Alkenyl, Y-($C_3$-$C_{12}$)-Cycloalkyl (mono-, bi- oder muiticyclisch), Phenyl, Y-Phenyl, Phenyl-($C_1$-$C_6$)-Alkyl, Phenyl-($C_1$-$C_4$)-Alkoxy, Biphenylyl, F, Cl, $CF_3$, Naphthyl, CN, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy, $-O-(CH_2)_{1-2}-O-$,

mit Y gleich Sauerstoff oder Schwefel,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)_r$-Kette mit r gleich 3 oder 4,

R(6) H, ($C_1$-$C_6$)-Alkyl, Y-($C_1$-$C_6$)-Alkyl, F, Cl, $CF_3$,

X Sauerstoff, Schwefel,

R(7) ($C_1$-$C_{12}$)-Alkyl, ($C_3$-$C_{12}$)-Cycloalkyl (mono-, bi- oder multicyclisch, wie Norbornyl oder Adamantyl), ($C_3$-$C_{12}$)-Alkenyl, Phenyl-thio-phenyl, Phenyl-oxy-phenyl, Biphenylyl, Phenyl-methyl-phenyl, Phenyl, Thienyl, wobei die genannten aromatischen und das heteroaromatische Ringsystem unsubstituiert oder ein- oder mehrfach substituiert sind durch F, Cl, Br, ($C_1$-$C_{18}$)-Alkyl, ($C_1$-$C_{18}$)-Alkoxy, ($C_2$-$C_{18}$)-Alkenyloxy, $CF_3$, $C_2F_5$, ($C_1$-$C_{18}$)-Alkylthio.

Besonders bevorzugt sind Verbindungen der Formel I, in denen bedeuten:

R(1): H, C(O)-($C_1$-$C_4$)-Alkyl, C(O)-phenyl

R(2): H, ($C_1$-$C_{15}$)-Alkyl, ($C_1$-$C_6$)-Alkyl-oxy-($C_1$-$C_6$)-Alkyl, ($C_1$-$C_6$)-Alkyl-thio-phenyl, ($C_1$-$C_6$)-Alkyl-oxy-phenyl, ($C_2$-$C_{15}$)-Alkenyl, ($C_3$-$C_{12}$)-Cycloalkyl (mono-, bi- oder multicyclisch, wie Norbornyl, Adamantyl, Dekahydronaphthyl), Phenyl-($C_1$-$C_6$)-Alkyl, Diphenyl-($C_1$-$C_6$-)-Alkyl, Phenyl, wobei die Phenylsysteme unsubstituiert oder ein- oder zweifach substituiert sind durch Substituenten aus der Gruppe F, Cl, ($C_1$-$C_8$)-Alkyl, ($C_3$-$C_6$)-Cycloalkyl, ($C_1$-$C_{10}$)-Alkoxy, ($C_1$-$C_4$)-Alkylendioxy, Phenoxy, Phenyl, Benzyl, Phenethyl, Thiophenyl, $CF_3$.

oder

R(2) Indol-3-yl-($C_1$-$C_4$)-Alkyl-, Thienyl- oder Thienylmethylrest, wobei der Thienylring unsubstituiert ist,

oder

R(2) Piperidin-4-yl, mit 1 bis 4 Methylgruppen substituiert und am Piperidin N-Atom ein Wasserstoff, eine Acetyl-, Phenyl- oder Benzylgruppe tragend,

R(3) Wasserstoff,

A $(C_1-C_{18})$-Alkyl, $(C_2-C_{18})$-Alkenyl oder eine Gruppe

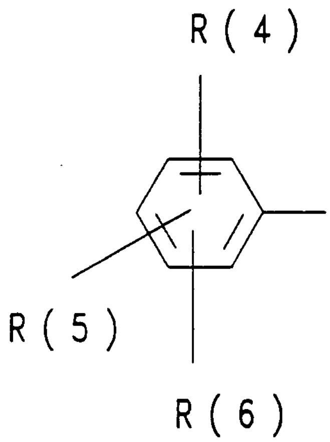

,

worin die Reste R(4), R(5) und R(6) die folgende Bedeutung haben:

R(4) H, $(C_1-C_{12})$-Alkyl, $(C_3-C_{12})$-Alkenyl, $(C_3-C_{12})$-Cycloalkyl (mono-, bi-oder multicyclisch), Y-$(C_1-C_{12})$-Alkyl, Y-$(C_3-C_{12})$-Alkenyl, Y-$(C_3-C_{12})$-Cycloalkyl (mono-, bi- oder multicyclisch), Phenyl, Y-Phenyl, Phenyl-$(C_1-C_6)$-Alkyl, Phenyl-$(C_1-C_4)$-Alkoxy, Biphenylyl, F, Cl, $CF_3$, wobei die Phenylsysteme unsubstituiert sind oder ein- oder zweifach substituiert durch F, Cl, $CF_3$, $(C_1-C_4)$-Alkyl, $(C_1-C_4)$-Alkoxy,

mit Y gleich Sauerstoff oder Schwefel,

R(5) wie R(4) definiert, wobei R(4) und R(5) gleich oder verschieden sind, oder

R(6) H, $(C_1-C_4)$-Alkyl, Y-$(C_1-C_4)$-Alkyl, F, Cl,

X Sauerstoff, Schwefel,

R(7) $(C_1-C_{10})$-Alkyl, $(C_3-C_{12})$-Cycloalkyl (mono-, bi- oder multicyclisch, wie Norbornyl oder Adamantyl), Phenyl-thio-phenyl, Phenyl-oxy-phenyl, Biphenylyl, Phenyl-methyl-phenyl, Phenyl, wobei die genannten aromatischen Ringsysteme unsubstituiert oder ein- oder mehrfach substituiert sind durch F, Cl, $(C_1-C_4)$-Alkyl, $(C_1-C_4)$-Alkoxy, $CF_3$, $(C_1-C_4)$-Alkylthio.

Ganz besonders bevorzugt sind Verbindungen der Formel I, in denen bedeuten:

R(1): H,

R(2): H, $(C_1-C_{15})$-Alkyl, $(C_1-C_6)$-Alkyl-oxy-$(C_1-C_6)$-Alkyl, $(C_1-C_6)$-Alkyl-thio-phenyl, $(C_1-C_6)$-Alkyl-oxy-phenyl, $(C_3-C_{12})$-Alkenyl, $(C_3-C_{10})$-Cycloalkyl (mono-, bi- oder multicyclisch, wie Norbornyl, Adamantyl, Dekahydronaphthyl), Phenyl-$(C_1-C_4)$-Alkyl, Diphenyl-$(C_1-C_6$-)-Alkyl, wobei die Phenylsysteme unsubstituiert oder ein- oder zweifach substituiert sind durch Substituenten aus der Gruppe F, Cl, $(C_1-C_8)$-Alkyl, $(C_3-C_6)$-Cycloalkyl, $(C_1-C_{10})$-Alkoxy, $(C_1-C_2)$-Alkylendioxy, Phenoxy, Phenyl, Benzyl, Phenethyl, Thiophenyl, $CF_3$.

oder

R(2) Indol-3-yl-$(C_1-C_4)$-Alkyl-, Thienyl- oder Thienylmethylrest, wobei der Thienylring unsubstituiert ist,

oder

R(2) Piperidin-4-yl, mit 1 bis 4 Methylgruppen substituiert und am Piperidin N-Atom ein Wasserstoff, eine Acetyl-, oder Benzylgruppe tragend,

R(3) Wasserstoff,

A eine Gruppe

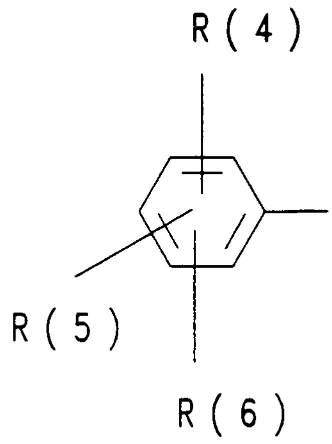

,

worin die Reste R(4), R(5) und R(6) die folgende Bedeutung haben:

R(4) H, ($C_1$-$C_{12}$)-Alkyl, ($C_3$-$C_{12}$)-Alkenyl, ($C_3$-$C_{12}$)-Cycloalkyl (mono-, bi-oder multicyclisch), Y-($C_1$-$C_{12}$)-Alkyl, Y-($C_3$-$C_{12}$)-Alkenyl, Y-($C_3$-$C_{12}$)-Cycloalkyl (mono-, bi- oder multicyclisch), Phenyl, Y-Phenyl, Phenyl-($C_1$-$C_6$)-Alkyl, Phenyl-($C_1$-$C_4$)-Alkoxy, F, Cl, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy,

mit Y gleich Sauerstoff oder Schwefel,

R(5) wie R(4) definiert, wobei R(4) und R(5) gleich oder verschieden sind,

R(6) H, ($C_1$-$C_4$)-Alkyl, Y-($C_1$-$C_4$)-Alkyl,

X Sauerstoff,

R(7) ($C_1$-$C_4$)-Alkyl, ($C_3$-$C_{12}$)-Cycloalkyl (mono-, bi- oder multicyclisch, wie Norbornyl oder Adamantyl), Phenyl-thio-phenyl, Phenyl-oxy-phenyl, Biphenylyl, Phenyl, wobei die genannten aromatischen Ringsysteme unsubstituiert oder ein- oder zweifach substituiert sind durch F, Cl, ($C_1$-$C_4$)-Alkyl, ($C_1$-$C_4$)-Alkoxy, $CF_3$, ($C_1$-$C_4$)-Alkylthio.

Racemate können nach üblichen Methoden in die Enantiomeren aufgetrennt werden, z. B. durch Salzbildung mit einer optisch aktiven Säure, Trennung der diastereomeren Salze und Freisetzung der reinen Enantiomeren mittels einer Base. Darüber hinaus lassen sich die Enantiomeren auch mittels Chromatographie an chiralen Phasen oder auf enzymatischem Wege trennen.

Die Erfindung betrifft weiterhin ein Verfahren zur Herstellung von Verbindungen der Formel (I), dadurch gekennzeichnet, daß man eine Verbindung der Formel (II),

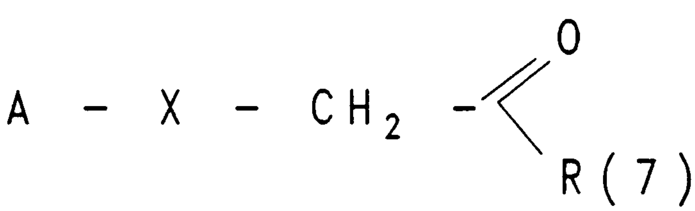

II

in welcher R(7), A und X, die genannten Bedeutungen haben, mit einem Schwefelylid der Formel III,

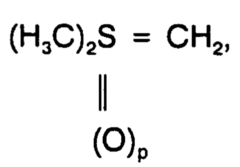

(III)

worin p Null oder 1 bedeutet,
zu einer Verbindung der Formel IV

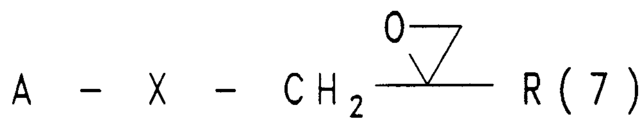

(IV)

umsetzt,
und anschließend die Verbindung IV mit einem Nucleophil der Formel MNR(2)R(3) umsetzt, in welcher R(2) und R(3) die genannten Bedeutungen haben und M Wasserstoff oder ein Metalläquivalent ist, wobei eine Verbindung I mit R(1) = H entsteht, die in Form der freien Base, eines Säureadditionssalzes oder eines physiologisch hydrolysierbaren Derivates isoliert wird.

Zur Herstellung der Verbindungen der Formel (I) wird in einem ersten Reaktionsschritt zur Herstellung der Verbindungen der Formel (IV) ein Keton der Formel (II), in der R(7), A und X die oben angegebenen Bedeutungen haben, mit einem Schwefelylid der Formel (III) in einem inerten Lösungsmittel, vorzugsweise Dimethylsulfoxid oder Tetrahydrofuran, oder in Gemischen von Dimethylsulfoxid mit anderen inerten Lösungsmitteln, z. B. Tetrahydrofuran, umgesetzt. Dabei ist bei Verwendung eines Schwefelylids der Formel (III), für das p = Null ist, ein Temperaturbereich von -10° bis 50 °C, vorzugsweise von 0 ° bis 30 °C zweckmäßig; bei Verwendung eines Schwefelylids der Formel (III), für das p = 1 ist, ein Temperaturbereich von 0 ° bis 80 °C, vorzugsweise von 20 ° bis 60 °C.

Die Umwandlung der Zwischenprodukte der Formel IV in die Endprodukte der Formel I mit R(1) = H erfolgt in einem zweiten Reaktionsschritt durch Umsetzung mit einer Verbindung der Formel MNR(2)R(3), in der R(2), R(3) und M die oben angegebenen Bedeutungen haben.

Diese Umsetzung erfolgt in einem Temperaturbereich von 20 - 160 °C, gegebenenfalls in einem inerten Lösungsmittel.

Geeignete Lösungsmittel sind beispielsweise N,N-Dimethylformamid, N,N-Dimethylacetamid, Dimethylsulfoxid, Sulfolan, N-Methylpyrrolidon-2, Dioxan, Tetrahydrofuran, Acetonitril, 4-Methyl-2-pentanon, Methanol, Ethanol, Isopropylalkohol, Propanol, Butanol, Pentanol, Tert.-butylalkohol, Methylglykol, Methylenchlorid oder ein aromatischer Kohlenwasserstoff wie Benzol, Chlorbenzol, Nitrobenzol, Toluol oder Xylol oder Wasser. Es können auch Gemische der beispielhaft genannten Lösungsmittel verwendet werden.

Die Umsetzung der Verbindungen der Formel II mit den Verbindungen der Formel III zu den Zwischenprodukten der Formel IV und Umsetzung dieser mit solchen der Formel MNR(2)R(3) zu Verbindungen der Formel I mit R(1) = H kann auch im Eintopfverfahren durchgeführt werden.

Hierzu stellt man zunächst, wie oben angegeben, durch Umsetzung einer Verbindung der Formel II mit einer Verbindung der Formel III eine Lösung einer Verbindung der Formel IV in einem der oben angegebenen inerten Lösungsmittel her. Anschließend gibt man zu dieser Lösung eine mindestens äquivalente Menge einer Verbindung der Formel HNR(2)R(3).

Verbindungen der Formel I, in denen R(1) die genannten Bedeutungen hat und nicht gleich Wasserstoff ist, werden hergestellt, indem man eine Verbindung der Formel mit R(1) = H mit einem entsprechenden Alkyl-, Alkenyl- oder Benzylhalogenid, vorzugsweise einem Chlorid, Bromid oder einem Tosylat oder Mesylat umsetzt, in Anwesenheit einer Base, in einem inerten Lösungsmittel, in einem Temperaturbereich von 0 - 100 °C. Als Lösungsmittel werden vorzugsweise diejenigen verwendet, die zur Umsetzung von Verbindungen der Formel IV mit M-NR(2)R(3) angegeben sind, als Basen eignen sich z. B. anorganische Basen wie Alkalimetall- oder Erdalkali-carbonate, -hydrogencarbonate, -hydroxide, - alkoholate oder - hydride, z. B. Natriumcarbonat, Natriumhydrogencarbonat, Kaliumcarbonat, Natriumhydroxid, Natriummethylat oder Natriumhydrid oder organische Basen wie tert.-Amine wie Triethylamin, Ethylmorpholin oder z. B. DBU, Imidazol oder Pyridin.

Verbindungen der Formel I, in denen R(1) die Bedeutung C(O)-($C_1$-$C_8$)-Alkyl oder -C(O)-phenyl hat, werden hergestellt, indem man eine Verbindung der Formel I mit R(1) gleich H mit einer Verbindung der Formel Va oder Vb

R(1) - Q
Va

R(1)-O-R(1)
Vb

umsetzt,
mit Q gleich Hydroxy, Halogen, wie Chlor oder Brom, einem Imidazolid. Dabei geht man am besten so vor, daß man eine Verbindung der Formel I mit R(1) gleich H in äquimolaren Mengen oder in bis zu einem 50-fachen Überschuß, gegebenenfalls in einem inerten, aprotischen Lösungsmittel, wie Chloroform, Dichlormethan, Tetrahydrofuran (THF) oder Dioxan, mit einer Verbindung der Formel Va bis zur Beendigung der Reaktion umsetzt, gegebenenfalls in Gegenwart einer Base, vorzugsweise Pyridin oder Triethylamin. Setzt man eine Verbindung der Formel Va mit Q gleich OH ein, so empfiehlt sich die Zugabe von Dicyclohexylcarbodiimid (DCC), gegebenenfalls in Anwesenheit eines Katalysators wie N,N-Dimethylaminopyridin (DMAP). Wird Vb (ein Säureanhydrid) verwendet

R(1) - O - R(1)  Vb,

so kann ohne oder mit einem Katalysator wie DMAP oder 4-Pyrrolidinopyridin (PPY) gearbeitet werden. Die Reaktionstemperaturen liegen dabei zwischen -70 °C und + 120 °C, vorzugsweise bei Verwendung eines Lösungsmittels zwischen dem Festpunkt und dem Siedepunkt des Lösungsmittels, insbesondere zwischen -30 und +60 °C. Die Reaktionszeiten betragen 1 bis 180 Stunden, bevorzugt 1 bis 48 Stunden, besonders bevorzugt 1 bis 8 Stunden.

Verbindungen der Formel I, die eine Thioethergruppe enthalten, können am Schwefel zu einem Sulfoxid oder Sulfon oxidiert werden. Dazu werden solche Verbindungen der Formel I mit einem Oxidationsmittel, z. B. Wasserstoffperoxid oder m-Chlorperbenzoesäure umgesetzt, in einem inerten Lösungsmittel, in einem Temperaturbereich von -10 bis 80 °C. Zur Herstellung von Sulfoxiden setzt man dazu ein Moläquivalent Oxidationsmittel ein, zur Herstellung von Sulfonen zwei Moläquivalente, gegebenenfalls auch einen Überschuß.

Als Lösungsmittel werden vorzugsweise diejenigen verwendet, die zur Umsetzung von Verbindungen der Formel IV mit M-NR(2)R(3) angegeben sind.

Die Erfindung betrifft weiterhin ein Verfahren zur Herstellung der Verbindungen der Formel I, nach welchem man eine Verbindung der Formel II

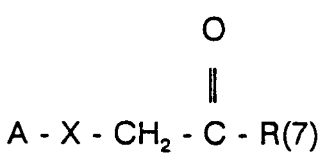

(II),

in welcher R(7), X und A die genannten Bedeutungen haben, mit einem Phosphorylid zu einer Verbindung der Formel VI

(VI)

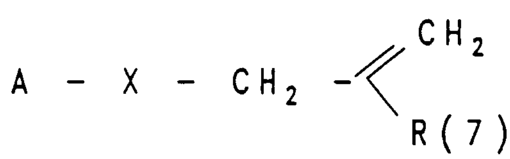

umsetzt,
und anschließend die Verbindung VI mit einem Peroxid zu einer Verbindung der Formel IV

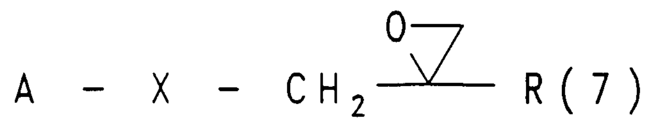

(IV)

umsetzt
und IV wie oben bereits erwähnt weiter mit einem Nucleophil der Formel M-NR(2)R(3) umsetzt, in welcher R(2), R(3) die genannten Bedeutungen haben und M Wasserstoff oder ein Metalläquivalent ist, wobei eine Verbindung I mit R(1) gleich Wasserstoff entsteht und, falls gewünscht, diese Verbindung I, alkyliert, alkenyliert, benzyliert oder verestert und gegebenenfalls am Schwefel einer Thioethergruppe zum Sulfoxid oder Sulfon oxidiert,
und gegebenenfalls in das Salz mit einer physiologisch verträglichen Säure oder in ein physiologisch hydrolysierbares Derivat überführt wird.

Zur Herstellung der Verbindungen der Formel I wird in einem ersten Reaktionsschritt zur Herstellung von Verbindungen der Formel V ein Keton der Formel II, in der A, X und R(7) und in die oben angegebenen Bedeutungen haben, mit einem Phosphorylid in einem inerten Lösungsmittel bei einer Temperatur von -60 bis +80 °C umgesetzt.

Das dabei verwendete Phosphorylid wird durch Umsetzung eines Methyltriarylphosphoniumsalzes, vorzugsweise Methyltriphenylphosphonium-chlorid, -bromid oder -jodid, mit einer starken Base in einem inerten Lösungsmittel hergestellt. Geeignete Basen sind z. B. Alkali- oder Erdalkalimetallhydride-, -amide oder -alkoholate, Natriumbistrimethylsilylamid oder Lithiumorganyle wie n-Butyl- oder Phenyl-Lithium.

Als Lösungsmittel werden diejenigen verwendet, die zur Umsetzung von Verbindungen der Formel IV mit M-NR(2)R(3) angegeben sind.

In einem zweiten Reaktionsschritt zur Herstellung von Verbindungen der Formel IV wird eine Verbindung der Formel VI mit einem Oxidationsmittel umgesetzt, in einem inerten Lösungsmittel, bei einer Temperatur von -10 bis +80 °C, gegebenenfalls in Anwesenheit einer Base und eines Nitrils, z. B. Benzonitril, oder Thionyl-bis-imidazol oder -triazol.

Geeignete Oxidationsmittel sind z. B. $H_2O_2$ oder Percarbonsäuren, wie z. B. Peressigsäure, Trifluorperessigsäure, Perbenzoesäure oder m-Chlorperbenzoesäure.

Als Lösungsmittel werden diejenigen verwendet, die zur Umsetzung von Verbindungen der Formel IV mit M-NR(2)R(3) angegeben sind.

In einem dritten Reaktionsschritt zur Herstellung von Verbindungen der Formel I mit R(1) = H aus Verbindungen der Formel IV verfährt man wie im vorstehenden Verfahren angegeben.

Diese Verbindungen der Formel I mit R(1) = H können, falls gewünscht, zu Verbindungen mit R(1) nicht gleich H alkyliert, alkenyliert, benzyliert oder verestert und oxidiert, und gegebenenfalls in das Salz mit einer physiologisch verträglichen Säure überführt werden, wie im vorstehenden Verfahren angegeben.

Herstellung der Ausgangsstoffe

Die Herstellung von Verbindungen der Formel II ist literaturbekannt. Beispielsweise wird ein α-Halogenketon der Formel VII:

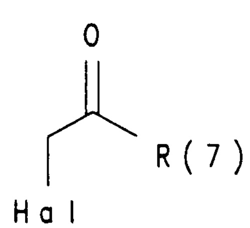

(VII)

mit einem Phenol oder Thiophenol unter Verwendung einer Base (z. B. $K_2CO_3$) in einem inerten Lösungsmittel wie Aceton umgesetzt.

Geeignete Basen sind beispielsweise Alkalimetall- oder Erdalkali-carbonate, -hydrogencarbonate, -hydroxide, oder -hydride wie z. B. Natriumcarbonat, Natriumhydrogencarbonat, Kaliumcarbonat, Natriumhydroxid oder Natriumhydrid.

Die Verbindungen der Formel VII sind entweder kommerziell erhältlich oder durch Halogenierung der entsprechenden Aryl-methyl-Ketone nach aus der Literatur bekannten Verfahren darstellbar.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung gegen und Behandlung von Pilzinfektionen beim Menschen und bei verschiedenen Säugetierarten.

Die Erfindung betrifft auch die Herstellung und Verwendung der Verbindungen des Typs I in Form der freien Basen oder eines Säureadditionssalzes als potente Hemmstoffe der Exoenzyme von Pilzen oder als Antimykotika.

Beispiele pharmazeutisch akzeptabler salzbildender Säuren sind anorganische Säuren wie Salzsäure, Bromwasserstoffsäure, Jodwasserstoffsäure, Schwefelsäure, Phosphorsäure oder Salpetersäure oder organische Säuren wie Malonsäure, Oxalsäure, Gluconsäure, Camphersulfonsäure, Benzolsulfonsäure, Essigsäure, Propionsäure, p-Toluolsulfonsäure oder Salicylsäure.

Die Verbindungen I weisen mindestens ein asymmetrisches C-Atom auf und können daher als Enantiomere und Diasteromere auftreten. Die Erfindung umfaßt sowohl die Herstellung und die Verwendung der reinen Isomeren als auch von deren Gemischen. Gemische von Diastereomeren können nach gebräuchlichen Methoden, z. B. durch selektive Kristallisation aus geeigneten Lösungsmitteln oder durch Chromatographie an Kieselgel oder Aluminiumoxid in die Komponenten aufgetrennt werden.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung gegen und Behandlung von Pilzinfektionen beim Menschen und bei verschiedenen Säugetierarten.

Die Verbindungen sind in vitro sehr gut wirksam gegen Hautpilze, wie z. B. Trichophyton mentagrophytes, Microsporum canis, Epidermophyton floccosum; gegen Schimmelpilze, wie z. B. Aspergillus niger oder gegen Hefen, wie z. B. Candida albicans, C. tropicalis, Torulopsis glabrata und Trichosporon cutanuem oder gegen Protozoen wie Trichomonas vaginalis oder T. fetus, oder auch gegen grampositive und gramnegative Bakterien.

Auch in vitro z. B. bei der experimentellen Nierencandidose der Maus, besitzen die Verbindungen nach oraler oder parenteraler Anwendung einen sehr guten systemischen Effekt, z. B. gegen Candida albicans. Hierbei wird von der Hefe Candida albicans insbesondere das Exoenzymsystem dergestalt beeinflußt, daß die Pathogenität der Erreger deutlich absinkt. Ebenso besteht ein sehr guter Effekt gegen verschiedene Erreger der Hautmykosen (z. B. Trichophyton mentagrophytes) am Meerschweinchen nach oraler, parenteraler oder lokaler Anwendung.

Als Indikationsgebiete in der Humanmedizin können beispielsweise genannt werden: Dermatomykosen und Systemmykosen durch Trichophyton mentagrophytes und andere Trichophytonarten, Mikrosporenarten, Epidermophyton floccosum, und biphasische Pilze sowie Schimmelpilze hervorgerufen.

Insbesondere werden tiefe Mykosen, die durch Candida albicans hervorgerufen werden, günstig beeinflußt, da hierbei ein Eindringen der Pilze in die Wirtszelle verhindert bzw. erschwert wird.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere Wirkstoffe enthalten oder die aus einem oder mehreren erfindungsgemäß verwendeten Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Ein Hemmstoff für die unterschiedlichen Phospholipasen von Candida albicans muß im Patienten überall dort in hinreichenden Konzentrationen vorliegen, wo der Pilz die Parenchyme besiedeln kann. Dieser Umstand setzt voraus, daß die entsprechenden Substanzen in einer Konzentration verabreicht werden müssen, die sich zuvor in Tierexperimenten als wirksam erwiesen hat.

Bei den schweren Krankheitsbildern der tiefen Candidose befinden sich die Patienten meist in einem sehr schlechten Allgemeinzustand. Hohes Fieber und weitere Erkrankungen sind häufig anzutreffen. Bei den Dosierungsvorgaben muß zwischen der prophylaktischen Gabe und der Therapie im nachgewiesenen Infektionsfall unterschieden werden. Bei der Prophylaxe kann von einem besseren Allgemeinzustand der Patienten ausgegangen werden, der eine orale Verabreichung ermöglicht. Hierbei können Tabletten, Lösungen, Gele oder Trockensaft zum Einsatz kommen. Bei den Formen mit nachgewiesenen tiefen Candidosen muß oft davon ausgegangen werden, daß eine geregelte orale Aufnahme der Wirkstoffe nicht immer gewährleistet ist. Hierfür kommen dann parenterale Anwendungsformen in Frage. Im Ausnahmefall

kann auch an eine subcutane Verabreichung gedacht werden.

Als Kandidaten für eine Prophylaxe kommen in erster Linie immunkomprimierte Patienten in Frage, die durch entsprechende medikamentöse Belastung oder durch körpereigene Immunprobleme in dieser Situation sind. Dies sind insbesondere Transplantationspatienten, Zuckerkranke und/oder adipöse Patienten, AIDS-Patienten, unter Chemotherapie stehende Patienten, Langzeitbeatmete usw.

Einige der Verbindungen zeigen Hemmungen der Phospholipase von Candida albicans, die weit unter der in vitro festgestellten minimalen inhibitorischen Konzentrationen der Wirkstoffe gegenüber Candida albicans liegen. Daher kann die Dosierung in vielen Fällen unter derjenigen liegen, die für eine reine antimykotische Therapie nötig wäre.

Die Wirkung im Patienten beruht darauf, daß die Wirkstoffe bei den Candida-Zellen, die sich in der Nähe der Parenchyme befinden, zwei unterschiedliche Effekte auslöst. Zum einen wird die Adhäsion der Hefezellen an die Körperzellen verhindert und zum anderen wird Candida albicans daran gehindert, die Körperzellen mit Keimschläuchen zu penetrieren.

Durch dieses duale Wirkungskonzept kann die Hefe ihre Pathogenität nicht zur vollen Ausprägung bringen. Allerdings muß erwähnt werden, das Candida albicans außer den Phospholipasen noch weitere Pathomechanismen wie z. B. die Protease besitzt. Die Anheftung an körpereigene Zellen ist jedoch der primäre Schritt zur Penetration. Da diese Anheftung durch Phospholipasehemmer verhindert wird, können die anderen Pathomechanismen nicht voll zur Geltung kommen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die prophylaktisch und therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäß verwendeten Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des Wirkstoffs oder der Wirkstoffe mit dem Trägerstoff oder den Trägerstoffen.

Zur vorliegenden Erfindung gehört die Verwendung der erfindungsgemäßen Wirkstoffe sowie den pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraparenteral und/oder rectal appliziert werden.

Im allgemeinen hat es sich sowohl in der Human- als auch in der Veterinärmedizin als vorteilhaft erwiesen, den oder die erfindungsgemäß verwendeten Wirkstoffe in Gesamtmengen von mindestens etwa 0,05, vorzugsweise 0,1, insbesondere 0,5 mg/kg Körpergewicht bis höchstens etwa 200, vorzugsweise bis 100, insbesondere bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen, bei tiefen Mykosen jedoch über wesentlich längere Zeiträume (bis zu 6 Wochen) verabreicht.

Es kann jedoch erforderlich sein, von den genannten Dosierungen abzuweichen, und zwar in Abhängigkeit von der Art und dem Körpergewicht des zu behandeinden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel 1

Synthese von 2-t-Butyl-1-(2,4-dichlorphenoxy)-3-isopropyl-amino-2-propanol (1.7)

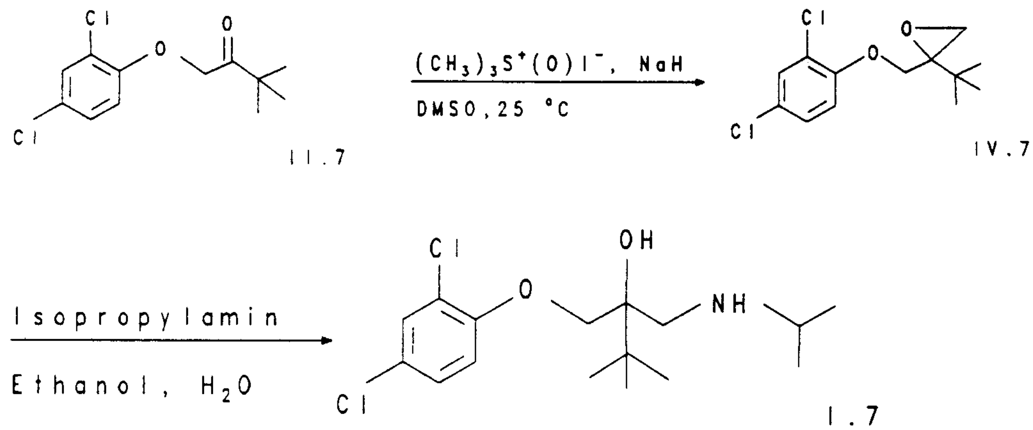


II.7 IV.7 I.7

(2 Stufen)

a) Herstellung des Oxirans IV.7

1,5 g Trimethylsulfoxoniumiodid (6,8 mmol) werden in 30 ml abs. DMSO gelöst und mit 0,195 g (6,5 mmol) Natriumhydrid (80 %ige Suspension in Mineralöl) versetzt und das Gemisch bis zum Ende der Gasentwicklung gerührt (ca. 30 min.). Anschließend wird bei 25 °C mit 1,51 g (5,8 mmol) 1-(2,4-Dichlorphenoxy)-3,3-dimethyl-2-butanon, gelöst in 5 ml abs. DMSO, versetzt und das Reaktionsgemisch 1 Stunde bei 25 °C und 2 Stunden bei 40 °C gerührt.

Zur Aufarbeitung wird das Reaktionsgemisch auf Wasser (400 ml) gegossen und die wäßrige Phase viermal mit jeweils 100 ml Dichlormethan extrahiert. Die vereinigten organischen Phasen werden viermal mit jeweils 100 ml Wasser gewaschen, über $Na_2SO_4$ getrocknet und nach Abfiltrieren des Trockenmittels unter reduziertem Druck am Rotationsverdampfer eingeengt.
Ausbeute: 1,24 g (78 %, 4,5 mmol, farbloses Öl).

Das Rohprodukt wird direkt, ohne weitere Reinigungsschritte, umgesetzt.

b) Umsetzung mit Isopropylamin

1 g des unter a) hergestellten Oxirans (3,63 mmol) werden in 30 ml Ethanol gelöst und mit 6,38 ml (4,43 g, 75 mmol) Isopropylamin sowie 1 ml Wasser versetzt. Das Reaktionsgemisch wird 12 Stunden unter Rückfluß erhitzt. Der Verlauf der Reaktion wird dünnschichtchromatographisch verfolgt (Kieselgelplatten, Laufmittel: Dichlormethan/Ethylacetat = 1/1).

Zur Aufarbeitung wird das Lösungsmittel unter reduziertem Druck am Rotationsverdampfer entfernt und das erhaltene Rohprodukt über Kieselgel chromatographiert (Kieselgel 60, Fa. Merck 0,043 - 0,060 mm, Laufmittel:
$CH_2Cl_2$/Methanol = 19/1).
Ausbeute: 0,972 g (2,9 mmol, 80 % d. Th.), farbloses Öl.

$R_F$ ($CH_2Cl_2$/Ethylacetat = 1 : 1 Kieselgelglasplatten) = 0,18.

Beispiel 2

Synthese von 3-N-Benzylamino-2-t-butyl-1-(4-heptyloxy-phenyl-oxy)-2-propanol (1.19)

(2 Stufen)

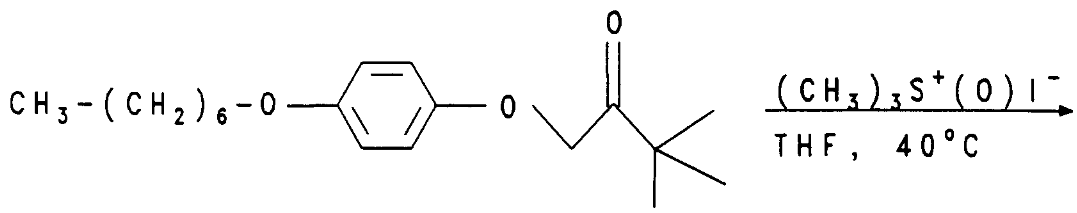


II.19

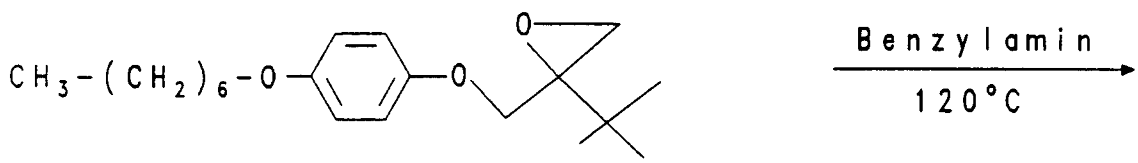


IV.19

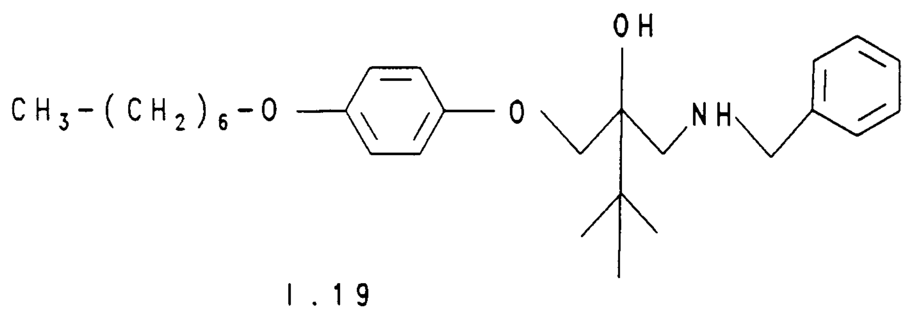

I.19

a) Herstellung des Oxirans IV.19

7,37 g (33,5 mmol) Trimethylsulfoxoniumiodid werden in 150 ml absolutem Tetrahydrofuran vorgelegt und unter $N_2$-Schutzgasatmosphäre portionsweise mit 0,88 g (29 mmol) Natriumhydrid (80 %ige Suspension in Paraffinöl) versetzt. Zur vollständigen Deprotonierung wird das Gemisch unter Rühren 2 h am Rückfluß erhitzt. Anschließend werden 7,67 g (25 mol) 3,3-Dimethyl-1-(4-heptyloxy-phenyl-oxy)-2-butanon, gelöst in 40 ml abs. THF, zugegeben und die Reaktionslösung 6 h auf 40° C erhitzt.

Zur Aufarbeitung wird das Reaktionsgemisch auf 400 ml Eiswasser gegossen und die wäßrige Phase nach Phasentrennung mit 4 x 100 ml Essigsäureethylester extrahiert. Die vereinigten organischen Phasen werden mit 4 x 100 ml Wasser gewaschen und über $Na_2SO_4$ getrocknet. Nach Abfiltrieren des Trockenmittels wird das Lösungsmittel unter reduziertem Druck entfernt.

Der gelbe feste Rückstand besitzt laut $^1$H-NMR-Spektren die angegebene Struktur und eine Reinheit von ca. 96 %.

Ausbeute 6,9 g (87 %), gelber Feststoff
$R_F$ (Kieselgel-Glasplatten, Laufmittel $CH_2Cl_2$): 0,42

b) Umsetzung des Oxirans IV.19 mit Benzylamin

1 g (3,14 mmol) des Oxirans aus a) werden in 6 ml Benzylamin gelöst und 2 h auf 120 °C erhitzt.

Nach Abdestillieren des größten Teils des Benzylamins mittels Kugelrohrdestillation unter Ölpumpenvakuum (0,1 mbar) wird über eine Kieselgelsäule chromatographiert (Laufmittel: $CH_2Cl_2$/MeOH = 40:1).

Das Reaktionsprodukt wird anschließend aus Heptan umkristallisiert.

Ausbeute: 1,002 g (2,3 mmol, 75 %)
Schmelzpunkt: 52 °C
Analog den Beispielen 1 und 2, jedoch unter Verwendung entsprechender Verbindungen der Formel IV und MNR(2)R(3) wurden auch in die Tabelle 1 aufgeführten Verbindungen der Formel I mit R(1) = H hergestellt. Die Beispiele 1 und 2 sind als 1.7 und 1.19 in Tabelle 1 enthalten.

1.1

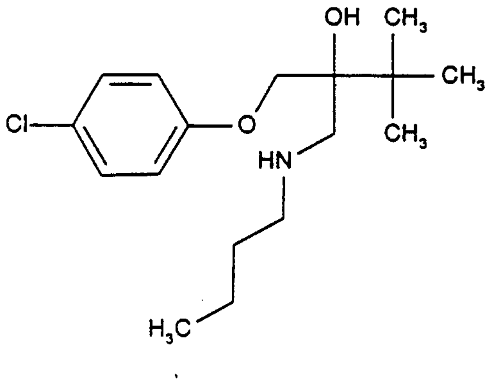

Fp.: Öl

EA: ber.: gef.: %

| | | |
|---|---|---|
| C | 65.06% | C 65.0 |
| H | 8.99% | H 8.9 |
| Cl | 11.30% | |
| N | 4.46% | N 4.5 |
| O | 10.19% | |

1.2

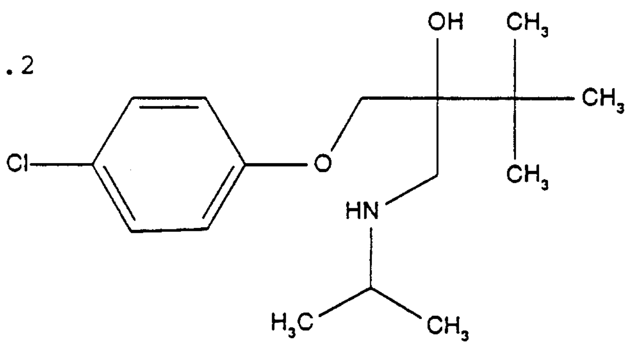

Fp.: Öl

EA: ber.: gef.:

| | | |
|---|---|---|
| C | 64.09% | 64.2 |
| H | 8.74% | 8.8 |
| Cl | 11.82% | |
| N | 4.67% | 4.8 |
| O | 10.67% | |

1.3

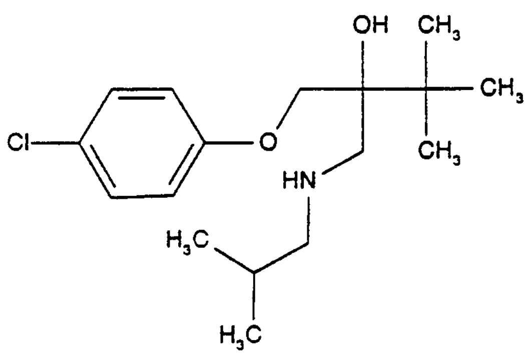

Fp.: Öl

EA: ber.: gef.:

| | | |
|---|---|---|
| C | 65.06% | 65.2 |
| H | 8.99% | 9.1 |
| Cl | 11.30% | |
| N | 4.46% | 4.6 |
| O | 10.19% | |

1.4

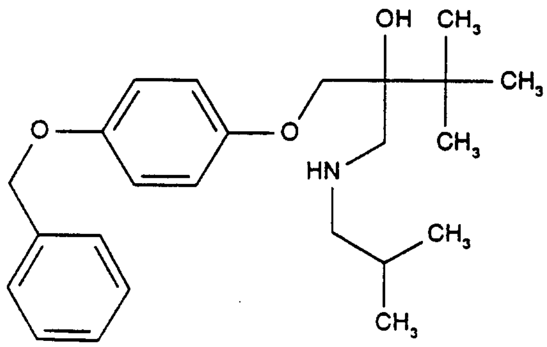

Fp.: Öl

| EA: | ber.: | gef.: |
|---|---|---|
| C | 74.77% | 74.7 |
| H | 9.15% | 9.3 |
| N | 3.63% | 3.7 |
| O | 12.45% | |

1.5

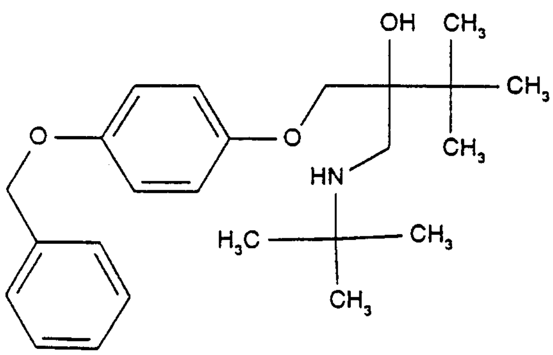

Fp.: Harz

| EA: | ber.: | gef.: |
|---|---|---|
| C | 74.77% | 74.8 |
| H | 9.15% | 9.2 |
| N | 3.63% | 3.8 |
| O | 12.45% | |

1.6

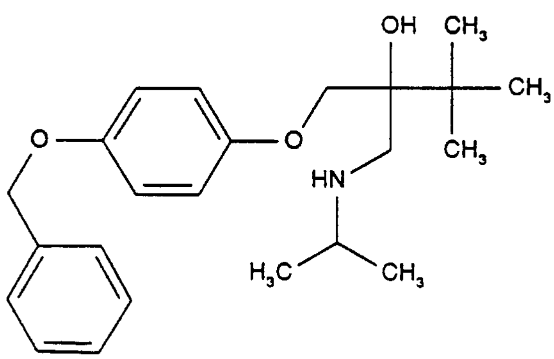

Fp.: Öl

| EA: | ber.: | gef.: |
|---|---|---|
| C | 74.36% | 74.5 |
| H | 8.95% | 9.0 |
| N | 3.77% | 3.7 |
| O | 12.92% | |

1.7

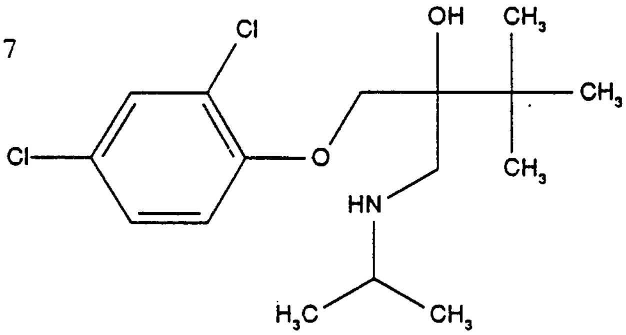

Fp.: Öl

EA:

| | ber.: | gef.: |
|---|---|---|
| C | 57.49% | 57.4 |
| H | 7.54% | 7.6 |
| Cl | 21.21% | |
| N | 4.19% | 4.3 |
| O | 9.57% | |

1.8

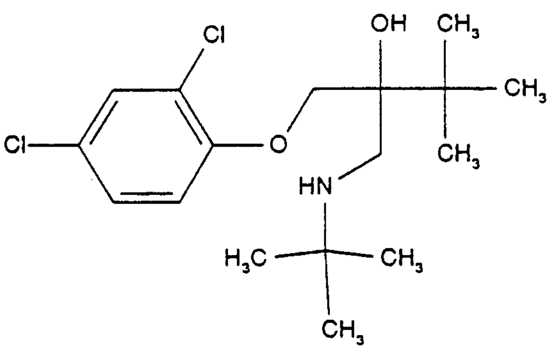

Fp.: Öl

EA:

| | ber.: | gef.: |
|---|---|---|
| C | 58.62% | 58.6 |
| H | 7.81% | 7.9 |
| Cl | 20.36% | |
| N | 4.02% | 4.1 |
| O | 9.19% | |

1.9

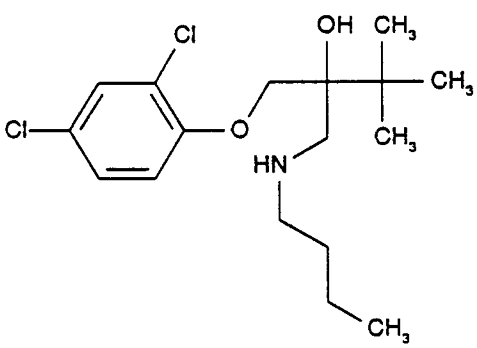

Fp.: Öl

EA:

| | ber.: | gef.: |
|---|---|---|
| C | 58.62% | 58.6 |
| H | 7.81% | 7.9 |
| Cl | 20.36% | |
| N | 4.02% | 4.2 |
| O | 9.19% | |

1.10

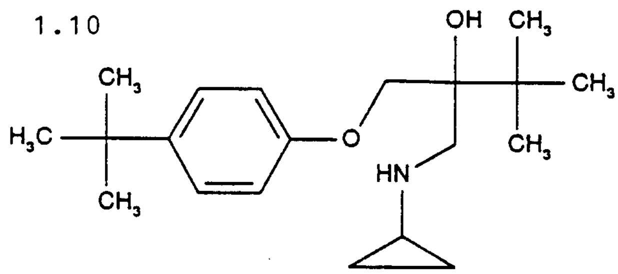

Fp.: Öl

EA:

| | ber.: | gef.: |
|---|---|---|
| C | 75.19% | 74.2 |
| H | 10.41% | 0.6 |
| N | 4.38% | 4.4 |
| O | 10.02% | |

1.11

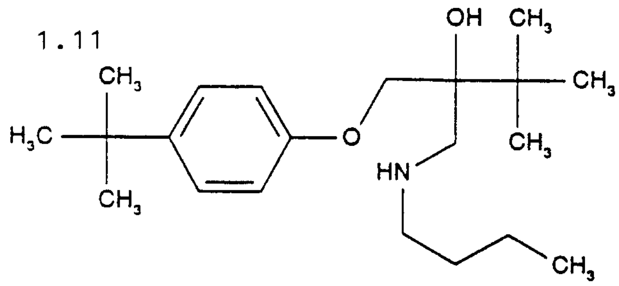

Fp.: Öl

EA:

| | ber.: | gef.: |
|---|---|---|
| C | 75.17% | 75.2 |
| H | 11.12% | 11.3 |
| N | 4.17% | 4.3 |
| O | 9.54% | |

1.12

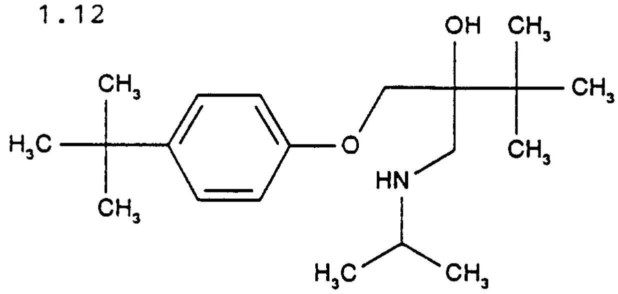

Fp.: Öl

EA:

| | ber.: | gef.: |
|---|---|---|
| C | 74.72% | 74.8 |
| H | 10.97% | 10.8 |
| N | 4.36% | 4.4 |
| O | 9.95% | |

Fp.: Öl

1.13

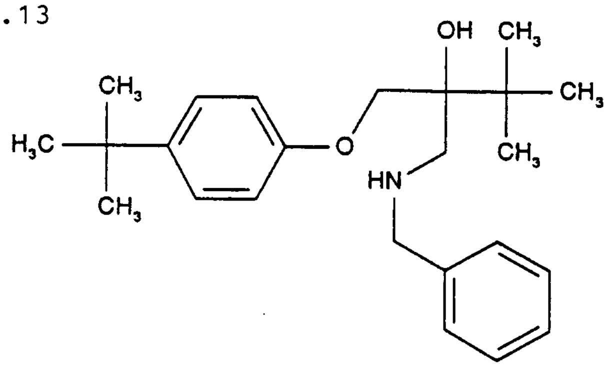

EA: ber.: gef.:

| | ber. | gef. |
|---|---|---|
| C | 78.00% | 78.2 |
| H | 9.55% | 9.6 |
| N | 3.79% | 3.8 |
| O | 8.66% | |

Fp.: Öl

1.14

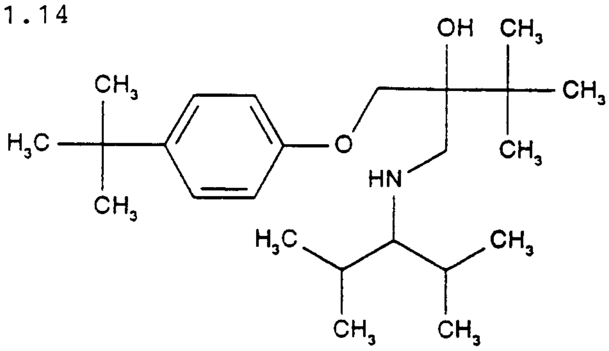

EA: ber.: gef.:

| | ber. | gef. |
|---|---|---|
| C | 76.34% | 76.4 |
| H | 11.48% | 11.6 |
| N | 3.71% | 3.9 |
| O | 8.47% | |

Fp.: Öl

1.15

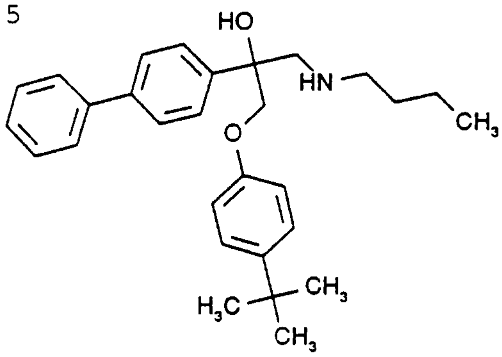

EA: ber.: gef.:

| | ber. | gef. |
|---|---|---|
| C | 80.70% | 80.8 |
| H | 8.64% | 8.6 |
| N | 3.25% | 3.3 |
| O | 7.41% | |

1.16

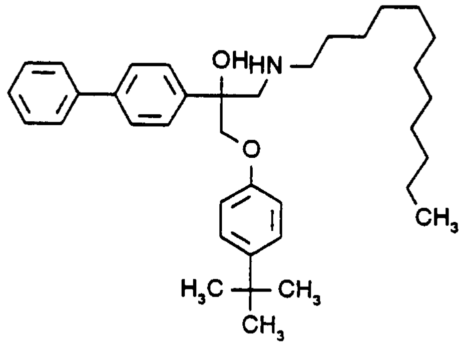

Fp.: Öl

EA: ber.: gef.:

| | ber. | gef. |
|---|---|---|
| C | 81.72% | 81.7 |
| H | 9.82% | 9.9 |
| N | 2.58% | 2.6 |
| O | 5.88% | |

A

1.17

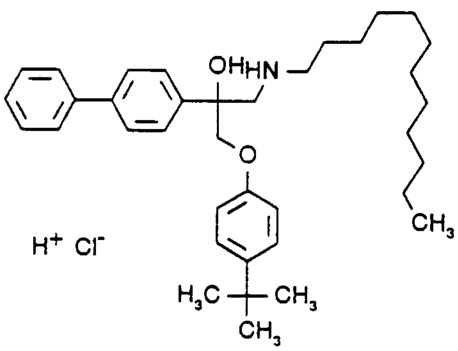

Fp.: Öl

EA: ber.: gef.:

| | ber. | gef. |
|---|---|---|
| C | 76.58% | 76.6 |
| H | 9.38% | 9.5 |
| Cl | 6.11% | |
| N | 2.41% | 2.7 |
| O | 5.51% | |

1.18

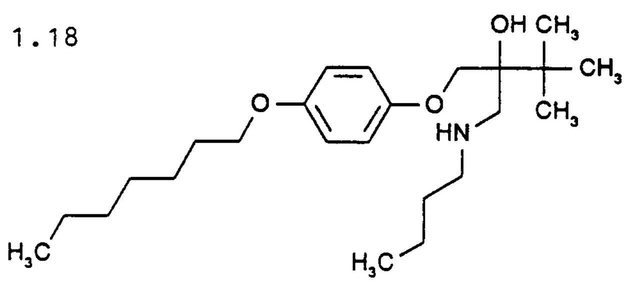

Fp.: Öl

EA: ber.: gef.:

| | ber. | gef. |
|---|---|---|
| C | 73.24% | 73.3 |
| H | 11.01% | 11.2 |
| N | 3.56% | 3.58 |
| O | 12.19% | |

Fp.: Öl

1.19

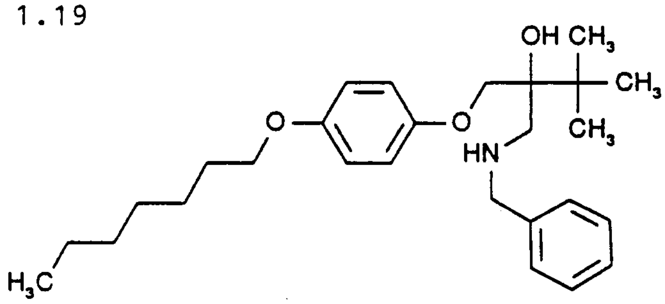

EA: ber.: gef.:

| | ber. | gef. |
|---|---|---|
| C | 75.84% | 75.9 |
| H | 9.66% | 9.8 |
| N | 3.28% | 3.3 |
| O | 11.22% | |

Fp.: Harz

1.20

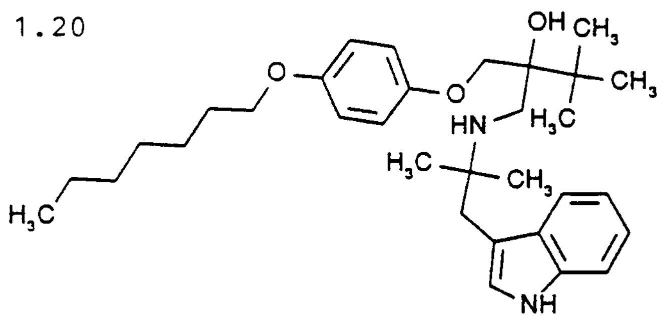

EA: ber.: gef.:

| | ber. | gef. |
|---|---|---|
| C | 75.55% | 75.3 |
| H | 9.51% | 9.5 |
| N | 5.51% | 5.6 |
| O | 9.43% | |

Fp.: Öl

1.21

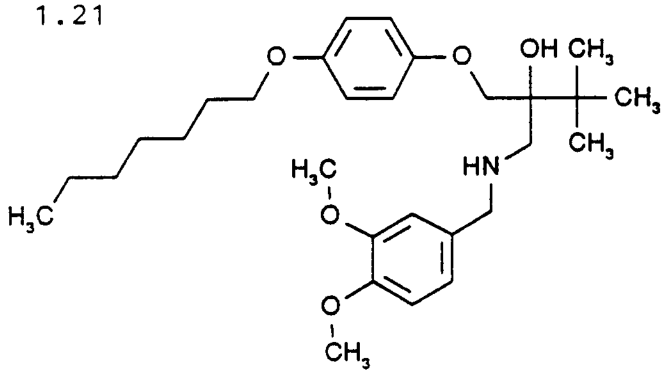

EA: ber.: gef.:

| | ber. | gef. |
|---|---|---|
| C | 71.42% | 71.4 |
| H | 9.30% | 9.3 |
| N | 2.87% | 3.0 |
| O | 16.40% | |

Fp.: Öl

1.22

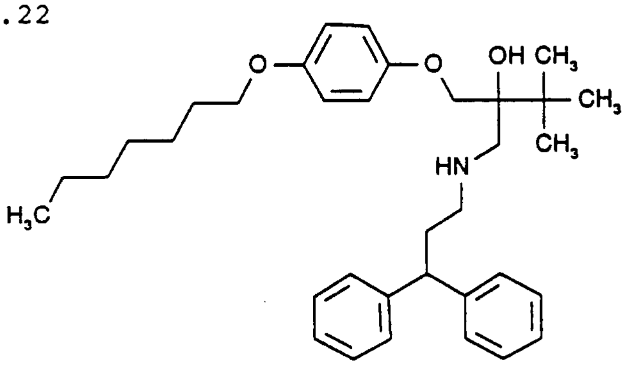

EA: ber.: gef.:

| | ber. | gef. |
|---|---|---|
| C | 79.05% | 79.3 |
| H | 9.29% | 9.1 |
| N | 2.63% | 2.6 |
| O | 9.03% | |

Fp.: Öl

1.23

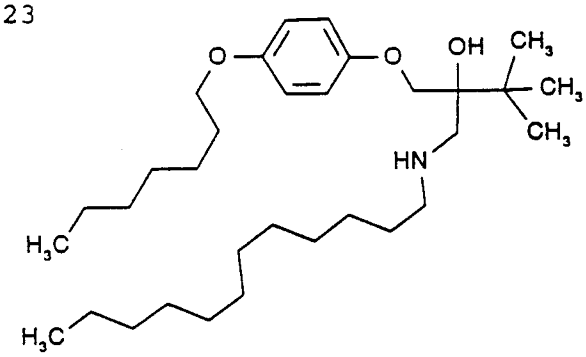

EA: ber.: gef.:

| | ber. | gef. |
|---|---|---|
| C | 75.99% | 75.7 |
| H | 11.76% | 11.8 |
| N | 2.77% | 2.9 |
| O | 9.49% | |

Fp.: Öl

1.24

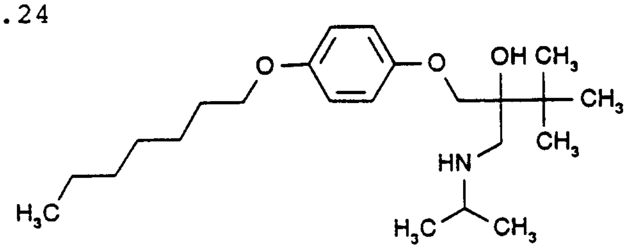

EA: ber.: gef.:

| | ber. | gef. |
|---|---|---|
| C | 72.78% | 73.0 |
| H | 10.89% | 10.7 |
| N | 3.69% | 3.6 |
| O | 12.64% | |

Fp.: Öl

1.25

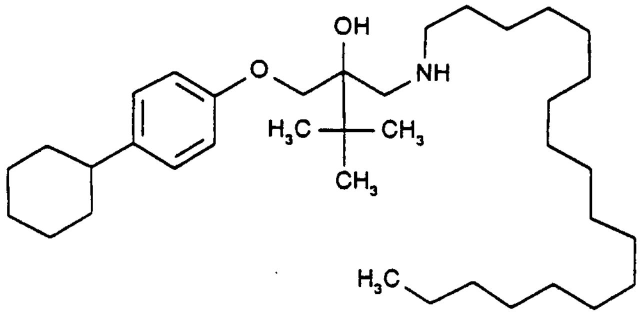

EA:

| | ber.: | gef.: |
|---|---|---|
| C | 79.65% | 79.6 |
| H | 12.10% | 12.3 |
| N | 2.51% | 2.6 |
| O | 5.74% | |

Fp.: Öl

1.26

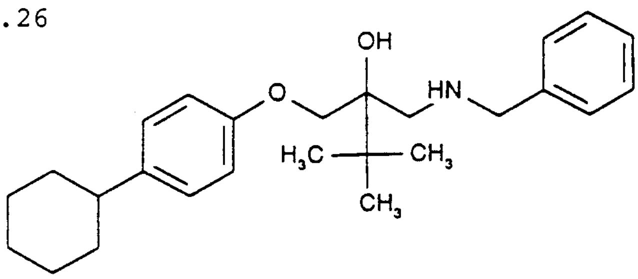

EA:

| | ber.: | gef.: |
|---|---|---|
| C | 78.94% | 78.9 |
| H | 9.43% | 9.4 |
| N | 3.54% | 3.7 |
| O | 8.09% | |

Fp.: Öl

1.27

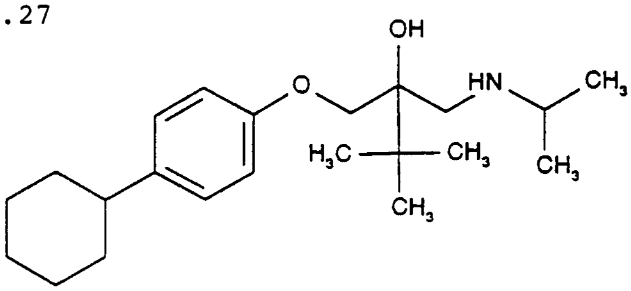

EA:

| | ber.: | gef.: |
|---|---|---|
| C | 76.03% | 76.2 |
| H | 10.73% | 10.9 |
| N | 4.03% | 4.0 |
| O | 9.21% | |

Fp.: Öl

1.28

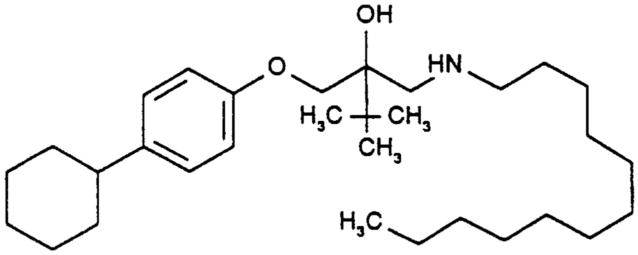

EA: ber.: gef.:

| | ber. | gef. |
|---|---|---|
| C | 78.59% | 78.7 |
| H | 11.70% | 11.6 |
| N | 2.96% | 3.0 |
| O | 6.75% | |

Fp.: Harz

1.29

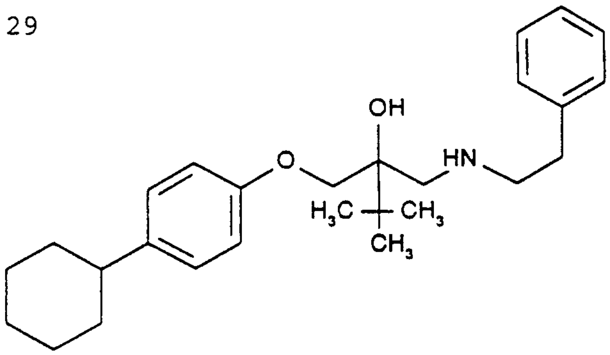

EA: ber.: gef.:

| | ber. | gef. |
|---|---|---|
| C | 79.17% | 79.2 |
| H | 9.60% | 9.8 |
| N | 3.42% | 3.4 |
| O | 7.81% | |

Fp.: Öl

1.30

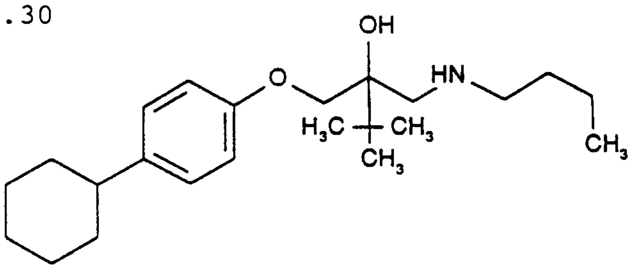

EA: ber.: gef.:

| | ber. | gef. |
|---|---|---|
| C | 76.40% | 76.4 |
| H | 10.87% | 10.9 |
| N | 3.87% | 3.9 |
| O | 8.85% | |

1.31

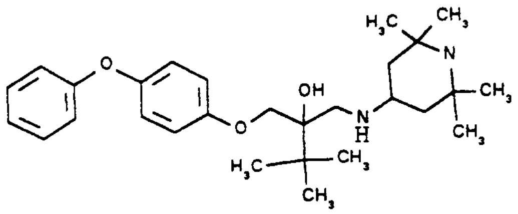

$C_{28}H_{42}N_2O_3$

| | gef.: | ber.: |
|---|---|---|
| C | 73,8 | 73,97 |
| H | 9,4 | 9,31 |
| N | 6,3 | 6,16 |
| O | | 10,56 |

1.32

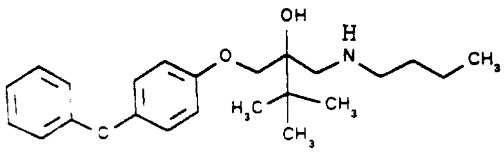

$C_{23}H_{33}NO_3$

| | gef.: | ber.: |
|---|---|---|
| C | 74,1 | 74,36 |
| H | 9,1 | 8,95 |
| N | 3,8 | 3,77 |
| O | | 12,92 |

1.33

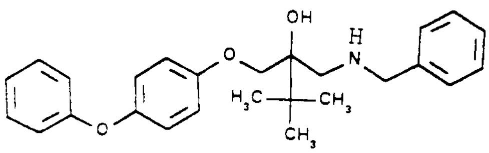

$C_{26}H_{31}NO_3$

| | gef.: | ber.: |
|---|---|---|
| C | 76,8 | 77,01 |
| H | 7,6 | 7,71 |
| N | 3,4 | 3,45 |
| O | | 11,84 |

1.34

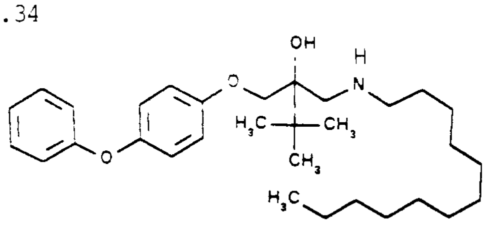

$C_{31}H_{49}NO_3$

| | gef.: | ber.: |
|---|---|---|
| C | 77,1 | 76,97 |
| H | 10,4 | 10,21 |
| N | 3,1 | 2,90 |
| O | | 9,92 |

1.35

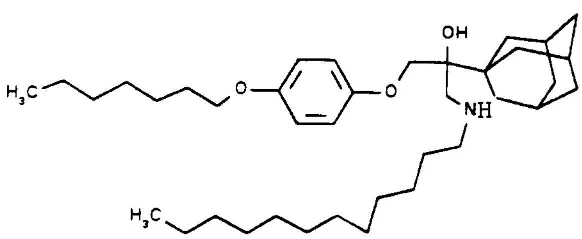

$C_{38}H_{65}NO_3$

| | gef.: | ber.: |
|---|---|---|
| C | 78,4 | 78,16 |
| H | 11,1 | 11,22 |
| N | 2,6 | 2,40 |
| O | | 8,22 |

1.36

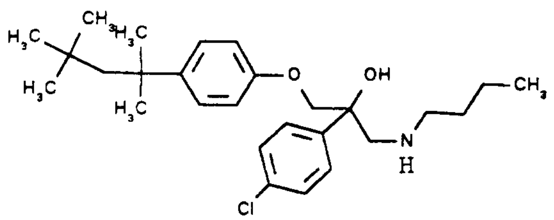

$C_{27}H_{40}ClNO_2$

| | gef.: | ber.: |
|---|---|---|
| C | 72,5 | 72,70 |
| H | 9,0 | 9,04 |
| N | 3,2 | 3,14 |
| O | | 7,17 |
| Cl | | 7,95 |

1.37

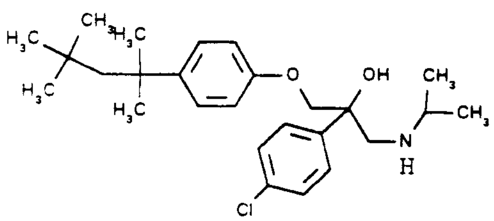

$C_{26}H_{38}ClNO_2$

| | gef.: | ber.: |
|---|---|---|
| C | 72,4 | 72,28 |
| H | 8,8 | 8,87 |
| N | 3,1 | 3,24 |
| O | | 7,40 |
| Cl | | 8,21 |

1.38

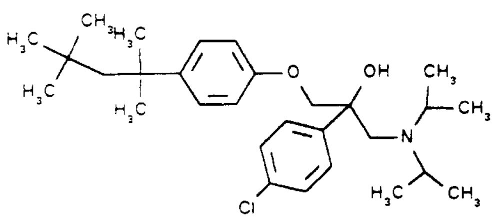

$C_{29}H_{44}ClNO_2$

| | gef.: | ber.: |
|---|---|---|
| C | 73,3 | 73,47 |
| H | 9,5 | 9,35 |
| N | 3,0 | 2,95 |
| O | | 6,75 |
| Cl | | 7,48 |

1.39

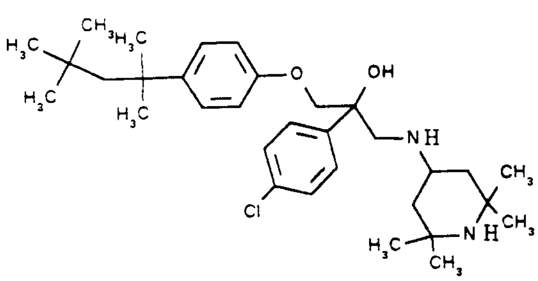

$C_{32}H_{49}ClN_2O_2$

| | gef.: | ber.: |
|---|---|---|
| C | 72,5 | 72,63 |
| H | 9,3 | 9,33 |
| N | 5,4 | 5,29 |
| O | | 6,05 |
| Cl | | 6,70 |

1.40

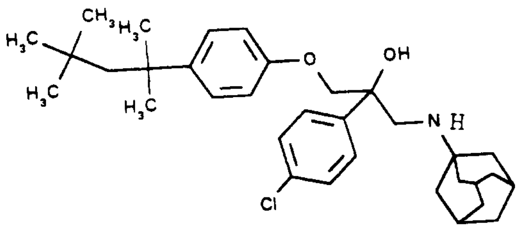

$C_{33}H_{46}ClNO_2$

| | gef.: | ber.: |
|---|---|---|
| C | 75,8 | 75,61 |
| H | 8,9 | 8,85 |
| N | 2,5 | 2,67 |
| O | | 6,10 |
| Cl | | 6,76 |

1.41

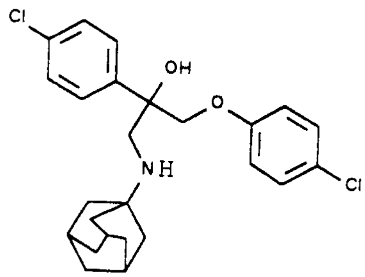

$C_{25}H_{29}Cl_2NO_2$

| | gef.: | ber.: |
|---|---|---|
| C | 67,4 | 67,26 |
| H | 6,6 | 6,55 |
| N | 3,2 | 3,14 |
| O | | 7,17 |
| Cl | | 15,88 |

1.42

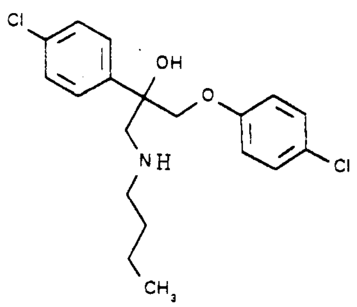

$C_{19}H_{23}Cl_2NO_2$

| | gef.: | ber.: |
|---|---|---|
| C | 61,8 | 61,96 |
| H | 6,4 | 6,29 |
| N | 3,9 | 3,80 |
| O | | 8,69 |
| Cl | | 19,25 |

1.43

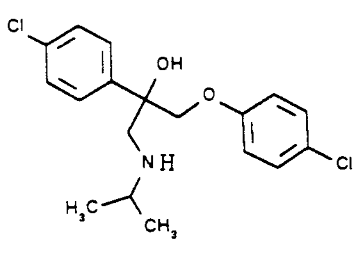

$C_{18}H_{21}Cl_2NO_2$

| | gef.: | ber.: |
|---|---|---|
| C | 60,8 | 61,03 |
| H | 6,0 | 5,97 |
| N | 3,9 | 3,95 |
| O | | 9,03 |
| Cl | | 20.01 |

1.44

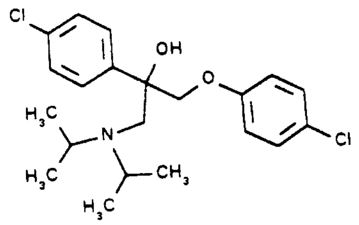

$C_{21}H_{27}Cl_2NO_2$

| | gef.: | ber.: |
|---|---|---|
| C | 63,5 | 63,64 |
| H | 6,8 | 6,87 |
| N | 3,4 | 3,53 |
| O | | 8,07 |
| Cl | | 17,89 |

1.45

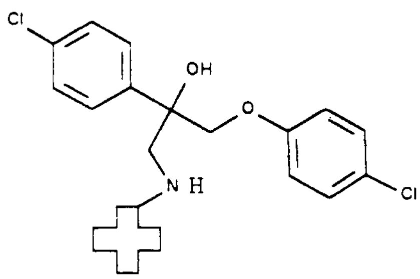

$C_{27}H_{37}Cl_2NO_2$

| | gef.: | ber.: |
|---|---|---|
| C | 67,9 | 67,78 |
| H | 7,8 | 7,79 |
| N | 3,0 | 2,93 |
| O | | 6,69 |
| Cl | | 14,82 |

1.46

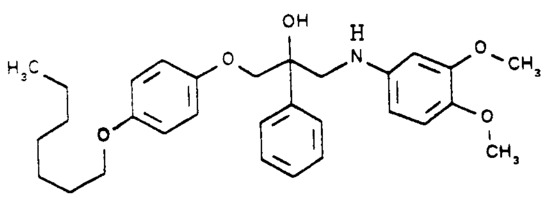

$C_{30}H_{39}NO_5$

| | gef.: | ber.: |
|---|---|---|
| C | 72,9 | 72,99 |
| H | 7,8 | 7,96 |
| N | | 2,84 |
| O | | 16,21 |

1.47

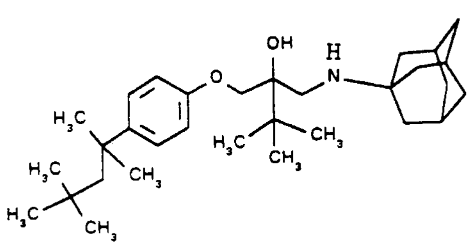

$C_{31}H_{51}NO_2$

| | gef.: | ber.: |
|---|---|---|
| C | 80,0 | 79,26 |
| H | 10,9 | 10,94 |
| N | 3,1 | 2,98 |
| O | | 6,81 |

1.48

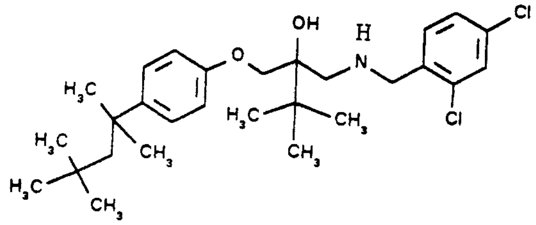

$C_{28}H_{41}Cl_2NO_2$

| | gef.: | ber.: |
|---|---|---|
| C | 67,8 | 68,00 |
| H | 8,4 | 8,36 |
| N | 2,8 | 2,83 |
| O | | 6,47 |

1.49

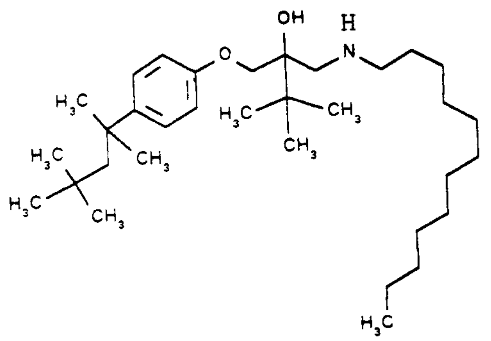

$C_{33}H_{61}NO_2$

| | gef.: | ber.: |
|---|---|---|
| C | 78,7 | 78,67 |
| H | 12,1 | 12,20 |
| N | 2,9 | 2,78 |
| O | | 6,35 |

1.50

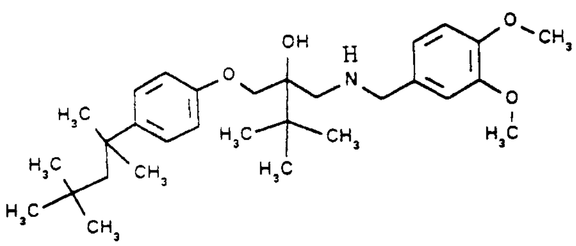

$C_{30}H_{47}NO_4$

| | gef.: | ber.: |
|---|---|---|
| C | 74,1 | 74,19 |
| H | 9,6 | 9,75 |
| N | 2,8 | 2,88 |
| O | | 13,18 |

1.51

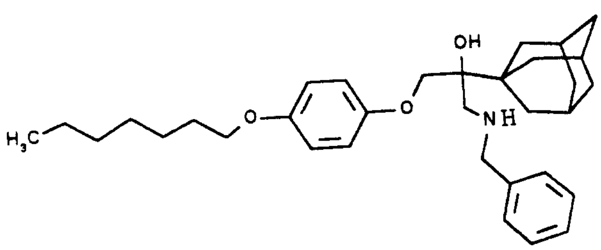

$C_{33}H_{47}NO_3$

| | gef.: | ber.: |
|---|---|---|
| C | 78,1 | 78,37 |
| H | 9,2 | 9,37 |
| N | 2,8 | 2,77 |
| O | | 9,49 |

1.52

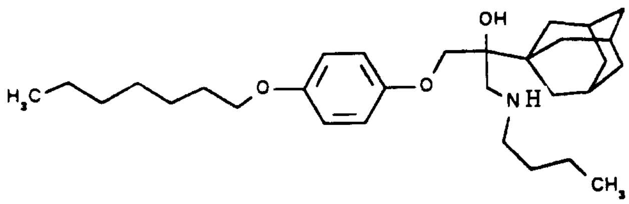

$C_{30}H_{49}NO_3$

| | gef.: | ber.: |
|---|---|---|
| C | 76,6 | 76,39 |
| H | 10,6 | 10,47 |
| N | 3,1 | 2,97 |
| O | | 10,17 |

1.53

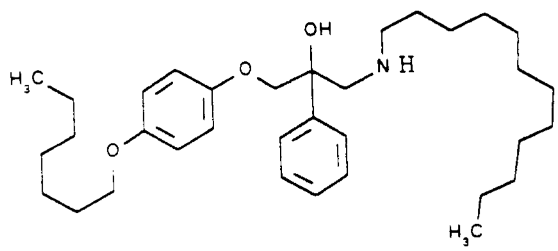

$C_{34}H_{55}NO_3$

| | gef.: | ber.: |
|---|---|---|
| C | 77,4 | 77,66 |
| H | 10,4 | 10,54 |
| N | 2,8 | 2,66 |
| O | | 9,13 |

Zur Feststellung der Enzymhemmung wurde eine Suspension von Candida albicans Blastokonidien (Stamm 200/175), wobei die Keimdichte photometrisch auf eine Extinktion von 0,5 (500 nm) eingestellt war, mit Präparatelösung bzw. zur Kontrolle mit Lösungsmittellösung vermischt und zwar

a) 100 µl Präparatelösung (Suspension)
+ 900 µl Keimsuspension
b) 100 µl Lösungsmittel
+ 900 µl Keimsuspension

Jeweils 5 µl der 30 min bei 21 °C inkubierten Blastokonidiensuspension wurden auf eine Agarplatte (Sabourand Agar mit Zusatz von 8 % Eigelb, 1 m NaCl, 5 mM $CaCl_2$) aufgetropft.

Die so beimpfte Platte wurde 3 Tage bei 37 °C bebrütet.

Die Auswertung erfolgte derart, daß

1. der Durchmesser (mm) der Candida albicans-Kolonie (behandelt und unbehandelt) sowie
2. der Gasamtdurchmesser von Kolonie und Trübungshof, welcher durch Exoenzyme verursacht wurde (behandelt und unbehandelt), bestimmt wurde.

Aus der Bestimmung des Quotienten von Präparate- und Kontrollgruppe ergab sich ein Wert, der als Maß für die Enzymaktivität anzusehen war.

Wie aus Tabelle 2 ersichtlich, hemmen die erfindungsgemäß verwendeten Verbindungen die freigesetzten Exoenzyme wesentlich stärker als Propranolol.

Propranolol wird in der Literatur (Pappu A.S. et al. in Biochem. Pharmacol. 34, 521 - 24, 1985) als wirksamste Substanz beschrieben, die gegenüber Phospholipase aus Leberzellen in einem entsprechenden in-vitro-Test Hemmwirkung zeigte.

Der Stand der Technik wird durch die erfindungsgemäß verwendeten Verbindungen I überraschenderweise deutlich übertroffen.

Tabelle 2

Prozentuale Hemmung der freigesetzten Exoenzyme von Candida albicans in vitro

| Präparat | Konzentration (µm/ml) | % Hemmung der Phospholipase |
|---|---|---|
| 1.5 | 100 | 100 |
| | 50 | 100 |
| 1.7 | 100 | 100 |
| | 100 | 100 |
| | 10 | 20 |
| 1.11 | 100 | 100 |
| | 100 | 100 |
| 1.15 | 100 | 100 |
| | 50 | 100 |
| | 10 | 100 |
| | 5 | 100 |
| 1.17 | 100 | 100 |
| | 50 | 100 |
| | 10 | 100 |
| | 5 | 100 |
| 1.18 | 100 | 100 |
| | 50 | 100 |

| | | |
|---|---|---|
| | 10 | 100 |
| | 5 | 100 |
| 1.19 | 100 | 100 |
| | 50 | 100 |
| | 10 | 100 |
| | 5 | 100 |
| 1.21 | 100 | 100 |
| | 50 | 100 |
| | 10 | 100 |
| 1.22 | 100 | 100 |
| | 50 | 100 |
| | 10 | 10 |
| 1.23 | 100 | 100 |
| | 50 | 100 |
| | 10 | 100 |
| | 5 | 100 |
| 1.24 | 100 | 100 |
| | 50 | 100 |
| | 10 | 100 |
| | 5 | 100 |
| 1.31 | 100 | 100 |
| | 50 | 100 |
| | 10 | 100 |
| | 5 | 7.7 |

| | | |
|---|---|---|
| 1.34 | 100 | 100 |
| | 50 | 100 |
| | 10 | 100 |
| | 5 | 33.3 |
| | 1 | 6.3 |
| 1.48 | 100 | 100 |
| | 50 | 100 |
| | 10 | 100 |
| | 5 | 100 |
| | 1 | 14.3 |
| 1.53 | 100 | 100 |
| | 50 | 100 |
| | 10 | 100 |
| | 5 | 100 |
| Propranolol | 100 | 30 |

Die pilzabtötende, antimykotische Wirksamkeit der Verbindungen wurde an einigen Beispielen (s. Tabelle 3) anhand der prozentualen Abtötung von Trichophyton mentagrophytes in vitro verifiziert (Standardpräparat Rilopirox als Vergleichssubstanz).

Tabelle 3

| Verbindung | | µg/ml |
|---|---|---|
| | 80 | 40 |
| 1.17 | 100 | 97,8 |
| Rilopirox | 100 | 100 |
| (Werte in %-Abtötung von Trichophyton mentagrophytes nach 14 h in aqua dest.) | | |

## Patentansprüche

**1.** Substituierte Aminopropane der Formel I

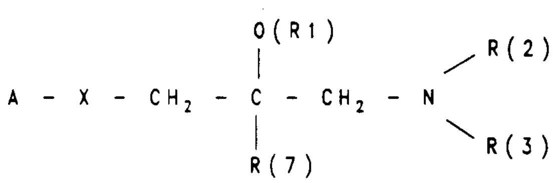

,

I

in welcher bedeuten:

R(1): H, ($C_1$- $C_{10}$)-Alkyl, ($C_2$-$C_{10}$)-Alkenyl, Benzyl, [unsubstituiert oder ein- oder mehrfach substituiert durch F, Cl, Br, $CF_3$, ($C_1$-$C_4$)-Alkyl, $OCH_3$, O-Phenyl oder Phenyl], C(O)-($C_1$-$C_8$)-Alkyl, C(O)-phenyl

R(2): H, ($C_1$-$C_{18}$)-Alkyl, ($C_1$-$C_6$)-Alkyl-oxy-($C_1$-$C_6$)-Alkyl, ($C_1$-$C_6$)-Alkyl-thio-phenyl, ($C_1$-$C_8$)-Alkyloxy-phenyl, ($C_2$-$C_{18}$)-Alkenyl, ($C_3$-$C_{12}$)-Cycloalkyl (mono-, bi- oder multicyclisch), Phenyl-($C_1$-$C_6$)-Alkyl, wobei die Alkylkette unsubstituiert oder mit OH ein- oder zweifach substituiert ist), Phenyl-($C_2$-$C_6$)-Alkenyl, Diphenyl-($C_1$-$C_6$-)-Alkyl, Phenyl, wobei die Phenylsysteme unsubstituiert oder ein- oder mehrfach substituiert sind durch Substituenten aus der Gruppe F, Cl, Br, ($C_1$-$C_{10}$)-Alkyl, ($C_3$-$C_{10}$)-Cycloalkyl, OH, SH, ($C_1$-$C_{10}$)-Alkoxy, ($C_1$-$C_4$)-Alkylendioxy, ($C_1$-$C_{10}$)-Alkylthio, Dimethylaminoethoxy, ($C_1$-$C_4$)-Alkoxycarbonylmethoxy, Phenoxy, Phenyl, Benzyl, Phenethyl, Thiophenyl, $C_aF_{2a+1}$ (mit a gleich 1 - 6).

oder

R(2) Indol-3-yl-($C_1$-$C_4$)-Alkyl-, Thienyl-, Thienylmethylrest, wobei der Thienylring unsubstituiert oder mit F, Cl, ($C_1$-$C_4$)-Alkyl, O($C_1$-$C_4$)-Alkyl substituiert ist,

oder

R(2) Piperidin-4-yl, unsubstituiert oder mit 1 bis 4 Methylgruppen substituiert und am Piperidin N-Atom ein Wasserstoff, eine Acetyl-, Phenyl- oder Benzylgruppe tragend,

R(3) ist wie R(2) definiert, wobei R(2) und R(3) jeweils gleiche oder verschiedene Bedeutung aufweisen, oder

R(2) bildet mit R(3) eine Kette $-(CH_2)_m-$ mit m gleich 4 - 6, in welcher eine $CH_2$-Gruppe durch Sauerstoff, Schwefel ersetzt sein kann,

A ($C_1$-$C_{18}$)-Alkyl, ($C_2$-$C_{18}$)-Alkenyl oder eine Gruppe

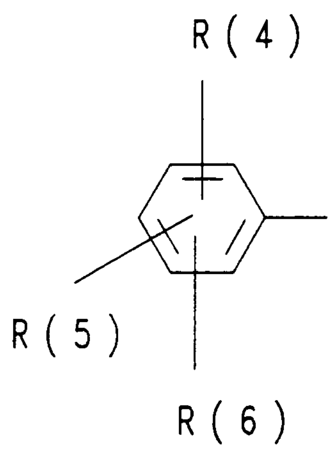

,

worin die Reste R(4), R(5) und R(6) die folgende Bedeutung haben:

R(4) H, ($C_1$-$C_{18}$)-Alkyl, ($C_2$-$C_{18}$)-Alkenyl, ($C_3$-$C_{20}$)-Cycloalkyl (mono-, bi- oder multicyclisch), Y-($C_1$-$C_{20}$)-Alkyl, Y-($C_2$-$C_{18}$)-Alkenyl, Y-($C_3$-$C_{20}$)-Cycloalkyl (mono-, bi- oder multicyclisch), Phenyl, Y-Phenyl, Phenyl-($C_1$-$C_6$)-Alkyl, Phenyl-($C_1$-$C_4$)-Alkoxy, Biphenylyl, F, Cl, Br, $C_aF_{2a+1}$ (mit a gleich 1 bis 8), $CCl_3$, YH, Naphthyl, CN, $NO_2$, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy, CN, $-O-(CH_2)_{1-2}-O-$,

mit Y gleich Sauerstoff oder Schwefel, SO, $SO_2$,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)_r$-Kette mit r gleich 3 oder 4, oder eine $O-(CH_2)_{1-2}-O$-Kette,

R(6) H, ($C_1$-$C_{15}$)-Alkyl, ($C_2$-$C_{15}$)-Alkenyl, Y-($C_1$-$C_{15}$)-Alkyl, Y-($C_2$-$C_{15}$)-Alkenyl, Phenyl, Y-Phenyl, Benzyl, Biphenylyl, F, Cl, Br, I, $C_aF_{2a+1}$ (mit a gleich 1 - 8), $CCl_3$, Naphthyl, YH,

X Sauerstoff, Schwefel,

R(7) ($C_1$-$C_{12}$)-Alkyl, ($C_3$-$C_{12}$)-Cycloalkyl (mono-, bi- oder multicyclisch, wie Norbornyl oder Adamantyl), ($C_3$-$C_{12}$)-Alkenyl, Phenyl-thio-phenyl, Phenyl-oxy-phenyl, Biphenylyl, Phenylmethyl-phenyl, Naphthyl, Phenyl, Thienyl, Pyridyl, wobei die genannten aromatischen und heteroaromatischen Ringsysteme unsubstituiert oder ein- oder mehrfach substituiert sind durch F, Cl, Br, ($C_1$-$C_{18}$)-Alkyl, ($C_1$-$C_{18}$)-Alkoxy, ($C_2$-$C_{18}$)-Alkenyloxy, $CF_3$, $C_2F_5$, CN, ($C_1$-

$C_{18}$)-Alkylthio.

**2.** Verbindung der Formel I nach Anspruch 1, in welcher bedeuten:

R(1): H, ($C_1$- $C_6$)-Alkyl, Benzyl, [unsubstituiert oder ein- oder zweifach substituiert durch F, Cl, Br, $CF_3$, ($C_1$-$C_4$)-Alkyl, $OCH_3$, O-Phenyl oder Phenyl], C(O)-($C_1$-$C_8$)-Alkyl, C(O)-phenyl

R(2): H, ($C_1$-$C_{18}$)-Alkyl, ($C_1$-$C_6$)-Alkyl-oxy-($C_1$-$C_8$)-Alkyl, ($C_1$-$C_6$)-Alkyl-thio-phenyl, ($C_1$-$C_6$)-Alkyl-oxy-phenyl, ($C_2$-$C_{18}$)-Alkenyl, ($C_3$-$C_{12}$)-Cycloalkyl (mono-, bi- oder multicyclisch), Phenyl-($C_1$-$C_6$)-Alkyl, wobei die Alkylkette unsubstituiert oder mit OH ein- oder zweifach substituiert ist), Phenyl-($C_2$-$C_6$)-Alkenyl, Diphenyl-($C_1$-$C_6$-)-Alkyl, Phenyl, wobei die Phenylsysteme unsubstituiert oder ein- oder mehrfach substituiert sind durch Substituenten aus der Gruppe F, Cl, Br, ($C_1$-$C_{10}$)-Alkyl, ($C_3$-$C_{10}$)-Cycloalkyl, ($C_1$-$C_{10}$)-Alkoxy, ($C_1$-$C_4$)-Alkylendioxy,($C_1$-$C_4$)-Alkoxycarbonylmethoxy, Phenoxy, Phenyl, Benzyl, Phenethyl, Thiophenyl, $CF_3$.

oder

R(2) Indol-3-yl-($C_1$-$C_4$)-Alkyl-, Thienyl- oder Thienylmethylrest, wobei der Thienylring unsubstituiert oder mit Cl, ($C_1$-$C_4$)-Alkyl, O($C_1$-$C_4$)-Alkyl substituiert ist,

oder

R(2) Piperidin-4-yl, unsubstituiert oder mit 1 bis 4 Methylgruppen substituiert und am Piperidin N-Atom ein Wasserstoff, eine Acetyl-, Phenyl- oder Benzylgruppe tragend,

R(3) ist wie R(2) definiert, wobei R(2) und R(3) jeweils gleiche oder verschiedene Bedeutung aufweisen, oder

A ($C_1$-$C_{18}$)-Alkyl, ($C_2$-$C_{18}$)-Alkenyl oder eine Gruppe

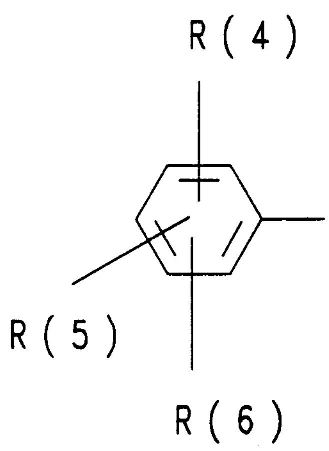

,

worin die Reste R(4), R(5) und R(6) die folgende Bedeutung haben:

R(4) H, ($C_1$-$C_{16}$)-Alkyl, ($C_3$-$C_{18}$)-Alkenyl, ($C_3$-$C_{12}$)-Cycloalkyl (mono-, bi- oder multicyclisch), Y-($C_1$-$C_{16}$)-Alkyl, Y-($C_3$-$C_{16}$)-Alkenyl, Y-($C_3$-$C_{12}$)-Cycloalkyl (mono-, bi- oder multicyclisch), Phenyl, Y-Phenyl, Phenyl-($C_1$-$C_6$)-Alkyl, Phenyl-($C_1$-$C_4$)-Alkoxy, Biphenylyl, F, Cl, $CF_3$, Naphthyl, CN, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy, -O-$(CH_2)_{1-2}$-O-,

mit Y gleich Sauerstoff oder Schwefel,

R(5) wie R(4) definiert, wobei R(4) und R(5) gleich oder verschieden sind, oder

R(4) bildet mit R(5), für den Fall, daß die Substituenten an benachbarten Positionen am Phenylring gebunden sind, gemeinsam eine $(CH_2)_r$-Kette mit r gleich 3 oder 4,

R(6) H, ($C_1$-$C_6$)-Alkyl, Y-($C_1$-$C_6$)-Alkyl, F, Cl, $CF_3$,

X Sauerstoff, Schwefel,

R(7) ($C_1$-$C_{12}$)-Alkyl, ($C_3$-$C_{12}$)-Cycloalkyl (mono-, bi- oder multicyclisch, wie Norbornyl oder Adamantyl), ($C_3$-$C_{12}$)-Alkenyl, Phenyl-thio-phenyl, Phenyl-oxy-phenyl, Biphenylyl, Phenyl-methyl-phenyl, Phenyl, Thienyl, wobei die genannten aromatischen und das heteroaromatische Ringsystem unsubstituiert oder ein- oder mehrfach substituiert sind durch F, Cl, Br, ($C_1$-$C_{18}$)-Alkyl, ($C_1$-$C_{18}$)-Alkoxy, ($C_2$-$C_{18}$)-Alkenyloxy, $CF_3$, $C_2F_5$, ($C_1$-$C_{18}$)-Alkylthio.

**3.** Verbindung der Formel I nach Anspruch 1, in welcher bedeuten:

R(1): H, C(O)-($C_1$-$C_4$)-Alkyl, C(O)-phenyl

R(2): H, ($C_1$-$C_{15}$)-Alkyl, ($C_1$-$C_6$)-Alkyl-oxy-($C_1$-$C_6$)-Alkyl, ($C_1$-$C_6$)-Alkyl-thio-phenyl, ($C_1$-$C_6$)-Alkyl-oxy-phenyl, ($C_2$-$C_{15}$)-Alkenyl, ($C_3$-$C_{12}$)-Cycloalkyl (mono-, bi- oder multicyclisch), Phenyl-($C_1$-$C_6$)-Alkyl, Diphenyl-($C_1$-$C_6$-)-Alkyl, Phenyl, wobei die Phenylsysteme unsubstituiert oder ein- oder zweifach substituiert sind durch Substituenten aus der Gruppe F, Cl, ($C_1$-$C_8$)-Alkyl, ($C_3$-$C_6$)-Cycloalkyl, ($C_1$-$C_{10}$)-Alkoxy, ($C_1$-$C_4$)-Alkylendioxy, Phenoxy, Phenyl, Benzyl, Phenethyl, Thiophenyl, $CF_3$.

oder

R(2) Indol-3-yl-($C_1$-$C_4$)-Alkyl-, Thienyl- oder Thienylmethylrest, wobei der Thienylring unsubstituiert ist,

oder

R(2) Piperidin-4-yl, mit 1 bis 4 Methylgruppen substituiert und am Piperidin N-Atom ein Wasserstoff, eine Acetyl-, Phenyl- oder Benzylgruppe tragend,

R(3) Wasserstoff,

A ($C_1$-$C_{18}$)-Alkyl, ($C_2$-$C_{18}$)-Alkenyl oder eine Gruppe

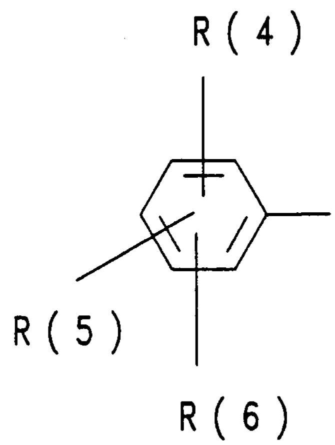

,

worin die Reste R(4), R(5) und R(6) die folgende Bedeutung haben:

R(4) H, ($C_1$-$C_{12}$)-Alkyl, ($C_3$-$C_{12}$)-Alkenyl, ($C_3$-$C_{12}$)-Cycloalkyl (mono-, bi- oder multicyclisch), Y-($C_1$-$C_{12}$)-Alkyl, Y-($C_3$-$C_{12}$)-Alkenyl, Y-($C_3$-$C_{12}$)-Cycloalky (mono-, bi- oder multicycisch), Phenyl, Y-Phenyl, Phenyl-($C_1$-$C_6$)-Alkyl, Phenyl-($C_1$-$C_4$)-Alkoxy, Biphenylyl, F, Cl, $CF_3$, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy,

mit Y gleich Sauerstoff oder Schwefel,

R(5) wie R(4) definiert, wobei R(4) und R(5) gleich oder verschieden sind, oder

R(6) H, ($C_1$-$C_4$)-Alkyl, Y-($C_1$-$C_4$)-Alkyl, F, Cl,

X Sauerstoff, Schwefel,

R(7) ($C_1$-$C_{10}$)-Alkyl, ($C_3$-$C_{12}$)-Cycloalkyl (mono-, bi- oder multicyclisch, wie Norbornyl oder Adamantyl), Phenyl-thio-phenyl, Phenyl-oxy-phenyl, Biphenylyl, Phenyl-methyl-phenyl, Phenyl, wobei die genannten aromatischen Ringsysteme unsubstituiert oder ein- oder mehrfach substituiert sind durch F, Cl, ($C_1$-$C_4$)-Alkyl, ($C_1$-$C_4$)-Alkoxy, $CF_3$, ($C_1$-$C_4$)-Alkylthio.

**4.** Verbindung der Formel I nach Anspruch 1, in welcher bedeuten:

R(1): H,

R(2): H, ($C_1$-$C_{15}$)-Alkyl, ($C_1$-$C_6$)-Alkyl-oxy-($C_1$-$C_8$)-Alkyl, ($C_1$-$C_6$)-Alkyl-thio-phenyl, ($C_1$-$C_6$)-Alkyl-oxy-phenyl, ($C_3$-$C_{12}$)-Alkenyl, ($C_3$-$C_{10}$)-Cycloalkyl (mono-, bi- oder multicyclisch), Phenyl-($C_1$-$C_4$)-Alkyl, Diphenyl-($C_1$-$C_6$-)-Alkyl, wobei die Phenylsysteme unsubstituiert oder ein- oder zweifach substituiert sind durch Substituenten aus der Gruppe F, Cl, ($C_1$-$C_8$)-Alkyl, ($C_3$-$C_6$)-Cycloalkyl, ($C_1$-$C_{10}$)-Alkoxy, ($C_1$-$C_2$)-Alkylendioxy, Phenoxy, Phenyl, Benzyl, Phenethyl, Thiophenyl, $CF_3$.

oder

R(2) Indol-3-yl-($C_1$-$C_4$)-Alkyl-, Thienyl- oder Thienylmethylrest, wobei der Thienylring unsubstituiert ist,

oder

R(2) Piperidin-4-yl, mit 1 bis 4 Methylgruppen substituiert und am Piperidin N-Atom ein Wasserstoff, eine Acetyl-, oder Benzylgruppe tragend,

R(3) Wasserstoff,

A eine Gruppe

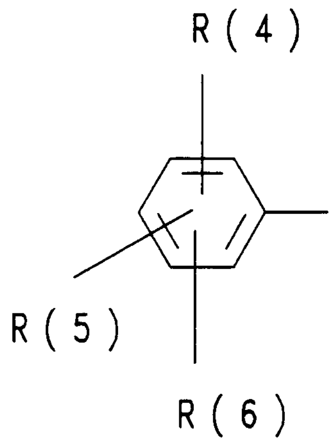

,

worin die Reste R(4), R(5) und R(6) die folgende Bedeutung haben:

R(4) H, ($C_1$-$C_{12}$)-Alkyl, ($C_3$-$C_{12}$)-Alkenyl, ($C_3$-$C_{12}$)-Cycloalkyl (mono-, bi- oder multicyclisch), Y-($C_1$-$C_{12}$)-Alkyl, Y-($C_3$-$C_{12}$)-Alkenyl, Y-($C_3$-$C_{12}$)-Cycloalkyl (mono-, bi- oder multicyclisch), Phenyl, Y-Phenyl, Phenyl-($C_1$-$C_6$)-Alkyl, Phenyl-($C_1$-$C_4$)-Alkoxy, F, Cl, wobei die Phenylsysteme unsubstituiert sind oder ein- oder zweifach substituiert durch F, Cl, $CF_3$, ($C_1$-$C_4$)-Alkyl, ($C_1$-$C_4$)-Alkoxy,

mit Y gleich Sauerstoff oder Schwefel,

R(5) wie R(4) definiert, wobei R(4) und R(5) gleich oder verschieden sind,

R(6) H, ($C_1$-$C_4$)-Alkyl, Y-($C_1$-$C_4$)-Alkyl,

X Sauerstoff,

R(7) ($C_1$-$C_4$)-Alkyl, ($C_3$-$C_{12}$)-Cycloalkyl (mono-, bi- oder multicyclisch, wie Norbornyl oder Adamantyl), Phenyl-thio-phenyl, Phenyl-oxy-phenyl, Biphenylyl, Phenyl, wobei die genannten aromatischen Ringsysteme unsubstituiert oder ein- oder zweifach substituiert sind durch F, Cl, ($C_1$-$C_4$)-Alkyl, ($C_1$-$C_4$)-Alkoxy, $CF_3$, ($C_1$-$C_4$)-Alkylthio.

**5.** Verfahren zur Herstellung von Verbindungen der Formel (I) nach Anspruch 1, dadurch gekennzeichnet, daß man eine Verbindung der Formel (II),

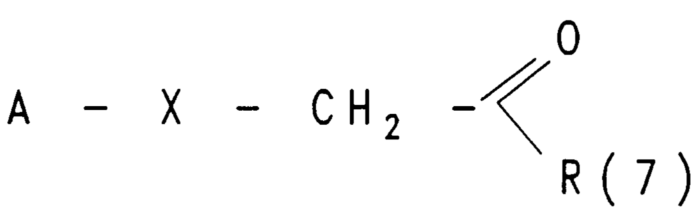

II

in welcher R(7), A und X, die in Anspruch 1 genannten Bedeutungen haben, mit einem Schwefelylid der Formel III,

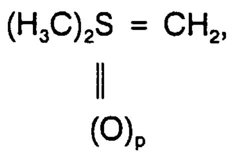

(III)

worin p Null oder 1 bedeutet, zu einer Verbindung der Formel IV

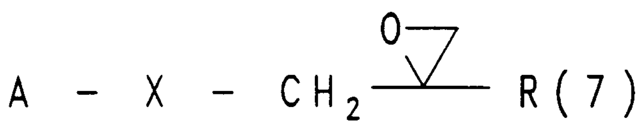

(IV)

umsetzt, und anschließend die Verbindung IV mit einem Nucleophil der Formel M-NR(2)R(3) umsetzt, in welcher R(2) und R(3) die genannten Bedeutungen haben und M Wasserstoff oder ein Metalläquivalent ist, wobei eine Verbindung I mit R(1) = H entsteht, die in Form der freien Base, eines Säureadditionssalzes oder eines physiologisch hydrolysierbaren Derivates isoliert wird.

**6.** Verbindung I nach Anspruch 1 zur Verwendung zur Behandlung und zur Prophylaxe von Pilzerkrankungen.

**7.** Verwendung einer Verbindung I nach Anspruch 1 zum Herstellen eines Medikaments zum Behandeln und zur Prophylaxe von Pilzerkrankungen.